(19) **Europäisches Patentamt / European Patent Office / Office européen des brevets**

(11) **EP 4 279 126 B1**

(12) # EUROPEAN PATENT SPECIFICATION

(45) Date of publication and mention of the grant of the patent:
**25.02.2026 Bulletin 2026/09**

(21) Application number: **23174171.1**

(22) Date of filing: **18.05.2023**

(51) International Patent Classification (IPC):
**A61N 5/10** $^{(2006.01)}$    **G06N 3/08** $^{(2023.01)}$
**G16H 20/40** $^{(2018.01)}$

(52) Cooperative Patent Classification (CPC):
**A61N 5/1037; A61B 5/113; A61B 5/7285;
A61N 5/1049; A61N 5/1067; G06N 3/045;
G06N 3/09; G06T 7/70; G16H 20/40; G16H 30/40;
G16H 40/63; G16H 50/20; G16H 50/70;**
A61B 5/7267; A61N 5/1068;    (Cont.)

(54) **TEMPORAL PREDICTION IN ANATOMIC POSITION MONITORING USING ARTIFICIAL INTELLIGENCE MODELING**

ZEITLICHE VORHERSAGE IN DER ANATOMISCHEN POSITIONSÜBERWACHUNG UNTER VERWENDUNG VON MODELLIERUNG VON KÜNSTLICHER INTELLIGENZ

PRÉDICTION TEMPORELLE DANS LA SURVEILLANCE DE POSITION ANATOMIQUE À L'AIDE D'UNE MODÉLISATION D'INTELLIGENCE ARTIFICIELLE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **19.05.2022 US 202263364999 P**

(43) Date of publication of application:
**22.11.2023 Bulletin 2023/47**

(73) Proprietor: **Elekta Limited**
**Montreal, Québec H3A 2J5 (CA)**

(72) Inventors:
• **VAZQUEZ ROMAGUERA, Liset**
**Montreal, H3A 2J5 (CA)**
• **NOVOSAD, Philip P.**
**Montreal, H3A 2J5 (CA)**

(74) Representative: **Hamer, Thomas Daniel**
**Kilburn & Strode LLP**
**Lacon London**
**84 Theobalds Road**
**London WC1X 8NL (GB)**

(56) References cited:
US-A1- 2021 046 327    US-A1- 2021 046 331
US-A1- 2021 192 279    US-A1- 2021 290 977
US-A1- 2021 304 866    US-A1- 2021 353 244
US-A1- 2022 020 189

• ISAKSSON MARCUS ET AL: "On using an adaptive neural network to predict lung tumor motion during respiration for radiotherapy applications", MEDICAL PHYSICS, AIP, MELVILLE, NY, US, vol. 32, no. 12, 29 November 2005 (2005-11-29), pages 3801 - 3809, XP012075244, ISSN: 0094-2405, DOI: 10.1118/1.2134958
• MEGUMI NAKAO ET AL: "IGCN: Image-to-graph Convolutional Network for 2D/3D Deformable Registration", ARXIV.ORG, CORNELL UNIVERSITY LIBRARY, 201 OLIN LIBRARY CORNELL UNIVERSITY ITHACA, NY 14853, 31 October 2021 (2021-10-31), XP091084201
• TONGHE WANG ET AL: "Medical Imaging Synthesis using Deep Learning and its Clinical Applications: A Review", ARXIV.ORG, CORNELL UNIVERSITY LIBRARY, 201 OLIN LIBRARY CORNELL UNIVERSITY ITHACA, NY 14853, 22 April 2020 (2020-04-22), XP081650135
• ROMAGUERA LISET V�ZQUEZ ET AL: "Prediction of in-plane organ deformation during free-breathing radiotherapy via discriminative spatial transformer networks", MEDICAL IMAGE ANALYSIS, OXFORD UNIVERSITY PRESS, OXOFRD, GB, vol. 64, 13 June 2020 (2020-06-13), XP086219044, ISSN: 1361-8415, [retrieved on 20200613], DOI: 10.1016/J.MEDIA.2020.101754

(52) Cooperative Patent Classification (CPC): (Cont.)
A61N 2005/1041; A61N 2005/1061;
G06T 2207/10016; G06T 2207/10081;
G06T 2207/10088; G06T 2207/10104;
G06T 2207/10108; G06T 2207/10116;
G06T 2207/10132; G06T 2207/20081;
G06T 2207/20084; G16H 50/30

**Description**

TECHNICAL FIELD

Embodiments of the present disclosure pertain generally to medical image and artificial intelligence processing techniques used in connection with a radiation therapy planning and treatment system. In particular, the present disclosure pertains to using machine learning technologies to estimate anatomic position and movement of a human subject (e.g., respiratory motion) for a radiation therapy session.

BACKGROUND

Radiation therapy (or "radiotherapy") can be used to treat cancers or other ailments in mammalian (e.g., human and animal) tissue. One such radiotherapy technique is provided using a Gamma Knife, by which a patient is irradiated by a large number of low-intensity gamma rays that converge with high intensity and high precision at a target (e.g., a tumor). Another such radiotherapy technique is provided using a linear accelerator (LINAC), whereby a tumor is irradiated by high-energy particles (e.g., electrons, protons, ions, high-energy photons, and the like). The placement and dose of the radiation beam must be accurately controlled to ensure the tumor receives the prescribed radiation, and the placement of the beam should be such as to minimize damage to the surrounding healthy tissue, often called the organ(s) at risk (OARs).

In radiotherapy, treatment planning is typically performed based on medical images of a patient and requires the delineation of target volumes and normal critical organs in the medical images. Then, during treatment delivery, additional images can be acquired to monitor patient motion (e.g. due to breathing). One challenge occurs with accurately tracking the various objects, such as a tumor, healthy tissue, or other aspects of patient anatomy when the patient is moving (e.g., breathing). This challenge arises because full three-dimensional patient motion occurring during radiotherapy treatment cannot be directly measured in real-time with current imaging hardware. On both conventional LINAC and MR-LINAC systems, the scan time for acquiring volumetric 3D images (e.g., 3D CBCT or 3D MRI images) is too long to capture respiratory motion of a human subject with sufficient temporal resolution, even with recent advances in artificial intelligence (AI) processing and compressed sensing.

Some imaging techniques have been developed to estimate the relative motion of a patient or an underlying an object contained in a specified region of interest, i.e. relative to a reference volume. For instance, some techniques attempt to estimate the underlying 3D patient motion from instantaneous partial measurements, using 2D images acquired in real-time. Other motion estimation techniques rely on detecting surface information that is indicative of patient movement, such as with sensors that are placed directly on a patient, or by tracking markers on a vest or a box affixed to the patient. However, these techniques assume that the surface information is correlated to internal patient state, which often is not accurate. As a result, anatomic position monitoring and motion estimates with existing techniques may be incomplete or incorrect.

US 2021/290977 A1 describes an irradiation control apparatus which includes processing circuitry. The processing circuitry obtains an MR image of a first time phase, which includes a tumor region of the patient, and estimates a position of the tumor region of the patient at a second time phase from the MR image of the first time phase, the second time phase being a predetermined time phase after the first time phase.

US 2021/046327 A1 describes systems and techniques used to estimate a real-time patient state during a radiotherapy treatment using a magnetic resonance linear accelerator.

OVERVIEW

The invention to which this European patent relates is defined in the appended claims. Embodiments, examples or aspects in the following disclosure which do not fall under the scope of the claims are presented for illustration purposes only and do not form part of the invention to which this European patent relates.

In some embodiments, methods, systems, and computer-readable mediums are provided for monitoring anatomic position and movement of a human subject during a radiotherapy treatment session. Such embodiments may include the use of a specially trained artificial intelligence (AI) model, such as a Transformer model, to generate an estimation of respiratory motion from prior breathing cycles of the patient observed with ongoing imaging. Such motion estimation may be used to
predict and continuously track the 3D position of a target region or anatomical area (e.g., region of interest, organs at risk, etc.) throughout the radiotherapy treatment session. The use of a Transformer model and similar self-attention AI models, in particular, allows the capture and evaluation of more historical data from longer spans of time, and even enables the prediction of movement along a larger horizon of time.

Based on the motion estimation, further operations may include performing a radiotherapy treatment with a radiotherapy machine, using the relative motion estimation of the region of interest. Performing the radiotherapy treatment may include operations such as: changing a position of a radiotherapy beam from the radiotherapy machine, based on the relative motion estimation; changing a shape of a radiotherapy beam from the radiotherapy machine, based on the relative motion estimation; or gating a radiotherapy beam (e.g., stopping an output of the radiotherapy beam, or starting an output of the radiotherapy beam), based on the relative motion estimation. Other variations or operations to radiotherapy planning and treatment may also be triggered or affected by the resulting motion estimation.

In some aspects, the techniques described herein relate to a computer-implemented method, non-transitory computer-readable storage medium with instructions, or a system configured for using a trained AI model for monitoring anatomic position of a human subject for a radiotherapy treatment session. Such techniques include: receiving position information corresponding to observed positions of a tracked anatomical area of a patient, the position information observed during the radiotherapy treatment session; providing the position information as an input to a trained model (e.g., a generative AI model), the trained model having been trained with temporal sequences of observed anatomical positions from training data; determining an estimated position of the tracked anatomical area of the patient at a future time, based on output of the trained model; and controlling the radiotherapy treatment session based on the estimated position of the tracked anatomical area of the patient.

In further aspects, the position information may be based on image data captured during the radiotherapy treatment session. The position information may be generated by extracting features from multiple images of the image data. In further aspects, the position information indicates a position of the tracked anatomical area of the patient in a 3D reference volume, wherein the estimated position is based on relative motion of the tracked anatomical area from translation or rotation in a coordinate space of the 3D reference volume.

In further aspects, the output of the trained model provides transformation parameters that indicate the relative motion of the tracked anatomical area of the patient relative to the 3D reference volume. The estimated position may be further determined based on monitoring signals captured during the radiotherapy treatment session from one or more sensors. Such monitoring signals may include a measurement of respiratory motion observed at a prior time of the radiotherapy treatment session.

In further aspects, the output of the trained model represents a prediction of respiratory motion to occur at the future time during the radiotherapy treatment session, wherein the estimated position of the tracked anatomical area of the patient corresponds to the prediction of respiratory motion. The observed positions of the tracked anatomical area of the patient may be captured during multiple observed breathing cycles, wherein the estimated position of the tracked anatomical area of the patient corresponds to multiple predicted breathing cycles.

In further aspects, the trained model may be re-trained at a plurality of update intervals during the radiotherapy treatment session, based on the observed positions of the tracked anatomical area of a patient. The trained model may be a transformer deep learning neural network model, trained as discussed below. The observed positions of anatomy used to train the model may be observed from the patient and multiple other human subjects.

In further aspects, the tracked anatomical area corresponds to at least one region of interest or at least one organ at risk defined for the radiotherapy treatment session. Controlling the radiotherapy treatment session may modify operation of a radiotherapy machine based on motion caused by the estimated position of the tracked anatomical area, including one or more of: changing a position of a radiotherapy beam from the radiotherapy machine; changing a shape of a radiotherapy beam from the radiotherapy machine; or gating a radiotherapy beam from the radiotherapy machine.

In further aspects, the techniques described herein relate to a computer-implemented method, non-transitory computer-readable storage medium with instructions, or a system configured for using a trained AI model for training an AI model for estimating anatomic position. Such techniques include: receiving training data providing temporal sequences of observed anatomical positions in a plurality of human subjects; training the AI model with the training data, the AI model configured to receive observed position data as input and provide estimated position data as output; and outputting the trained AI model for use in a radiotherapy treatment session. In an example, the AI model is a generative AI model that is trained as a transformer deep learning neural network model.

In further aspects, the output of the trained AI model includes transformation parameters that indicate relative motion of a tracked anatomical area indicated in the observed position data. The relative motion of the tracked anatomical area may correspond to one or more predicted breathing cycles. The input of the trained AI model may include observed position data representing observed positions of the tracked anatomical area that are captured over multiple observed breathing cycles.

In further aspects, the techniques include extracting features from image data for the observed anatomical positions in the plurality of human subjects, wherein the training data includes time-ordered sequences of the extracted features.

In further aspects, the techniques include re-training the AI model during the radiotherapy treatment session, based on additional sequential temporal data corresponding to observed positions of a tracked anatomical area of a patient of the radiotherapy treatment session.

The above overview is intended to provide an overview of subject matter of the present patent application. It is not intended to provide an exclusive or exhaustive explanation of the inventive subject matter. The detailed description is included to provide further information about the present patent application.

BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example but not by way of limitation, various embodiments discussed in the present document.

FIG. 1 illustrates a radiotherapy system, according to some examples.
FIG. 2A illustrates a radiation therapy system having radiation therapy output configured to provide a therapy beam, according to some examples.
FIG. 2B illustrates a system including a combined radiation therapy system and an imaging system, such as a cone beam computed tomography (CBCT) imaging system, according to some examples.
FIG. 3 illustrates a partially cut-away view of a system including a combined radiation therapy system and an imaging system, such as a nuclear magnetic resonance (MR) imaging (MRI) system, according to some examples.
FIG. 4 illustrates anatomic position monitoring operations, according to some examples.
FIG. 5 illustrates a configuration of an attention-based Transformer AI model for temporal prediction in anatomic position monitoring, according to some examples.
FIG. 6 illustrates a treatment workflow for performing anatomic position monitoring, using results of a trained AI model, according to some examples.
FIGS. 7A and 7B illustrate respective flowcharts for a method of training a generative AI model for estimating anatomic position, and a method of monitoring anatomic position of a human subject using the trained generative AI model, according to some examples.
FIG. 8 illustrates a flowchart for a method performed by an image processing computing system in performing training and treatment workflows, according to some examples.
FIG. 9 illustrates an exemplary block diagram of a machine on which one or more of the methods as discussed herein can be implemented.

DETAILED DESCRIPTION

In the following detailed description, reference is made to the accompanying drawings which form a part hereof, and which is shown by way of illustration-specific embodiments in which the present disclosure may be practiced. These embodiments, which are also referred to herein as "examples," are described in sufficient detail to enable those skilled in the art to practice the disclosure, and it is to be understood that the embodiments may be combined, or that other embodiments may be utilized and that structural, logical and electrical changes may be made without departing from the scope of the present disclosure. The following detailed description is, therefore, not to be taken in a limiting sense, and the scope of the present disclosure is defined by the appended claims and their equivalents.

The following discusses various implementations of an anatomic position monitoring (APM) technique usable in radiotherapy or radiosurgery applications. In an example, this APM technique may be used to track, in real-time, the motion of an object contained in a particular anatomical area such as in a specified region of interest. This APM technique includes the analysis of real-time data, such as position data determined from 2D images captured on an ongoing basis, with a trained artificial intelligence (AI) model. In an example, this trained AI model can be used to infer (predict) the true 3D motion of a specified region of interest (and related anatomical structure), which can be used to cause various radiotherapy treatment adaptations.

In conventional radiotherapy techniques, larger margins are often used to account for motion such as motion caused by breathing or other minor patient movements. With image guided radiation therapy (IGRT) with APM it is possible to obtain more accurate targeting, therefore margins can be reduced. IGRT may use a combination of computed tomography (CT) imaging, cone beam CT (CBCT), magnetic resonance (MR) imaging, positron-emission tomography (PET) imaging, or the like to obtain a 3D or 4D image of a patient prior to irradiation. Localizing the motion of the human subject during the actual irradiation treatment delivery (intra-fraction motion) may allow reduction of additional treatment margins that would otherwise be used to encompass motion.

APM involves a variety of imaging-based computations and methods, including those using fast cine imaging, to continuously track the 3D position of a target region throughout treatment. One promising application of APM is intra-fractional motion compensation. Examples of intra-fraction motion compensation include automatic gating, whereby the radiation delivery is enabled only when the target region and the delivery beam sufficiently overlap, and adaptive multi-leaf collimator (MLC) tracking, whereby the delivery beam dynamically adapts to the moving target using estimated positions. Both approaches allow for margin reduction while ensuring target coverage, but require real-time position estimates to accurately adapt the beam to the target.

Prior methods for generating real-time relative motion estimates have involved limited analysis of 2D images during radiotherapy treatment. Some of these methods have attempted to use 2D-to-2D or 2D-to-3D image registrations in order to estimate movement in three dimensions. However, in practice, image acquisition, position estimation via APM, and subsequent beam modulation/tracking each have a specific latency, resulting in a significant cumulative system latency. If this cumulative latency is large enough, temporal prediction methods are required to compensate. Some prior approaches have also attempted to develop regression models to predict motion and provide some level of real-time monitoring for radiotherapy delivery. Some prior approaches have also attempted use of recurrent neural networks, such as such as Long Short-Term Memory (LSTM) and Gated Recurrent Units (GRU) models, to consider more or different types of data inputs. However, these models are often very difficult or time-consuming to train, and often can only consider a limited amount of temporal data as an input.

In contrast to these technical limitations, the following APM methods and implementations provide use of an artificial intelligence model which considers one or more sequence(s) of prior temporal data, such as real-time position data. One such AI model which is particularly suited to this setting is a Transformer deep learning model, which uses a "self-attention" mechanism to consider larger sets of input data.

Recently, the Transformer model has been applied to demonstrate breakthroughs in many natural language processing (NLP) tasks, demonstrating state-of-the-art performance for representation learning. Similar to natural language, respiratory signals also present a sequential dependency, which can be leveraged as prior knowledge to regress future values. In particular, a Transformer model can be adapted to obtain direct access to an entire input sequence, allowing large input sequences (e.g., multiple breathing cycles) to be directly processed. In one aspect of the approaches discussed herein, the Transformer model is specifically adapted and applied for respiratory motion prediction, which then can be used for position estimation and related radiotherapy treatment effects.

The technical benefits of the following APM techniques include improved accuracy in the delivery of radiotherapy treatment dosage from a radiotherapy machine, the enhanced evaluation of prior real-world data (in real-time) to produce or perform more accurate radiotherapy machine treatment plans, improved AI model training and optimization, and reduced latency in computation and data processing operations. Such technical benefits may result in many apparent medical treatment benefits, including improved accuracy of radiotherapy treatment, reduced exposure to unintended radiation, reduction of side-effects, more accurate compliance with a radiology treatment plan, and the like.

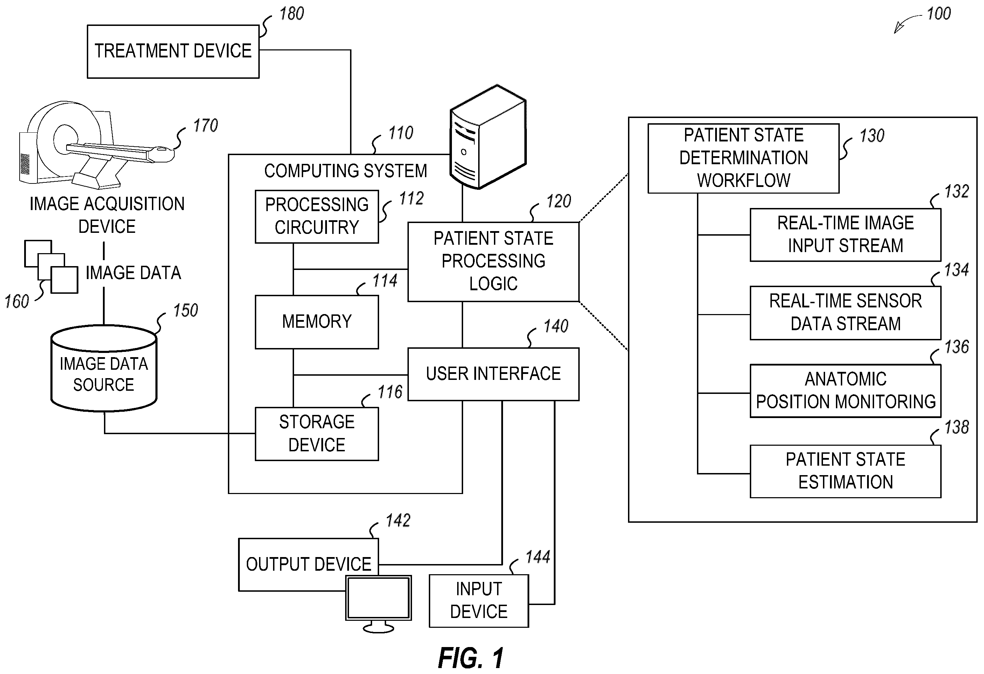

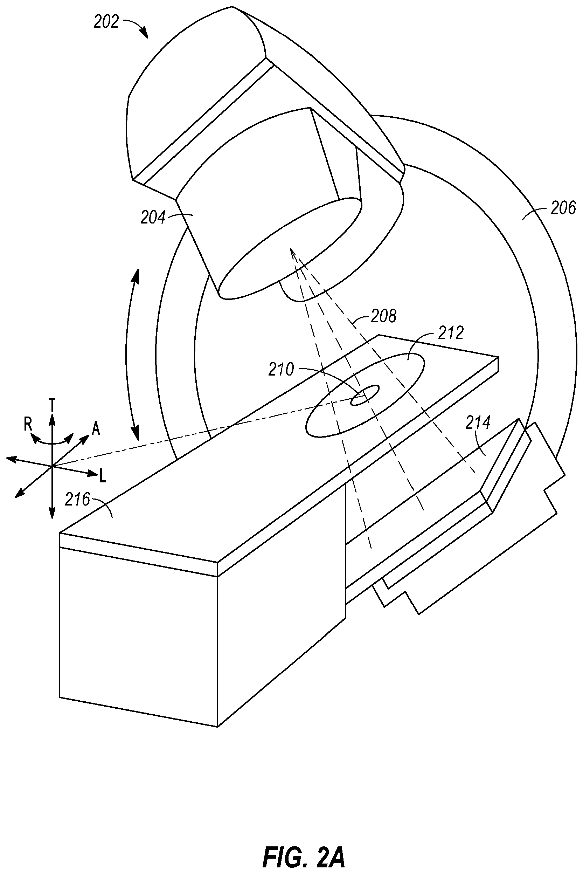

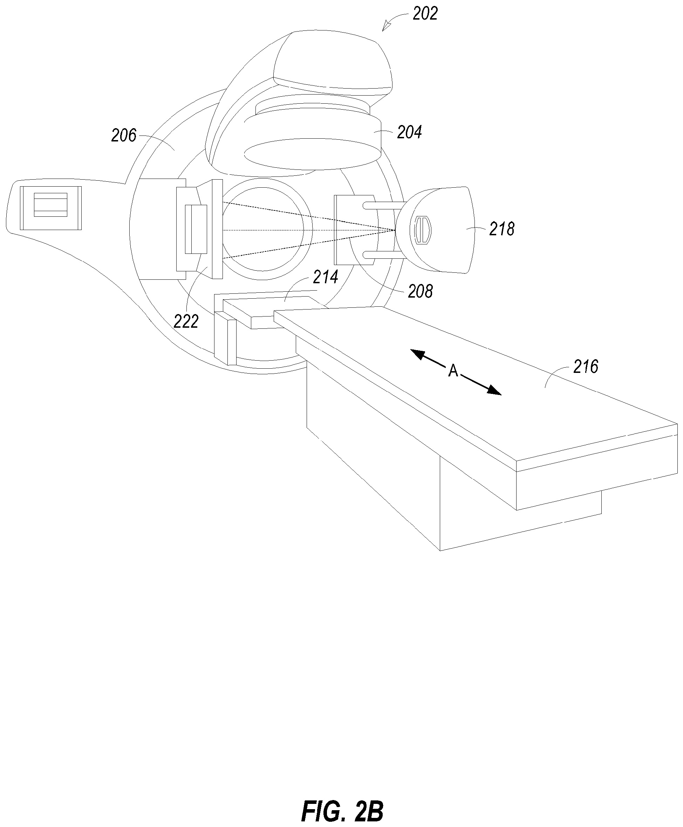

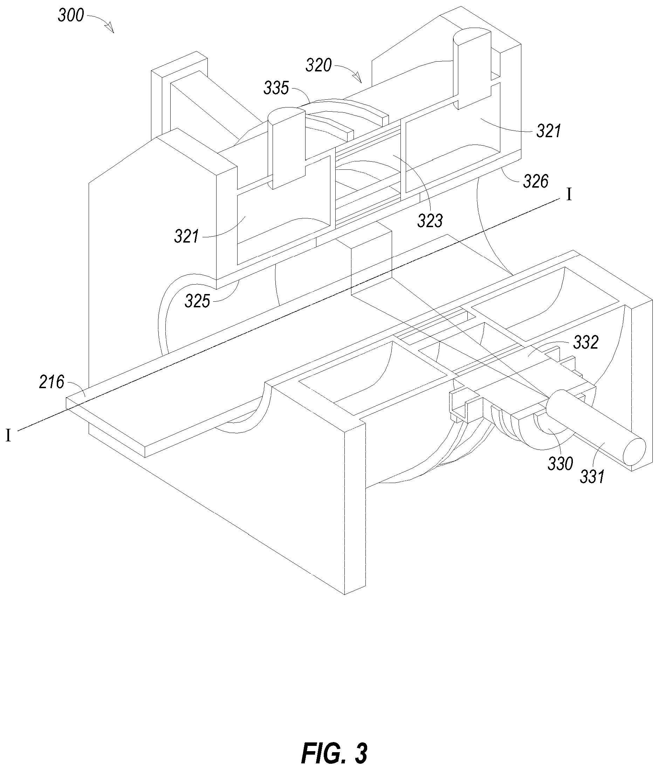

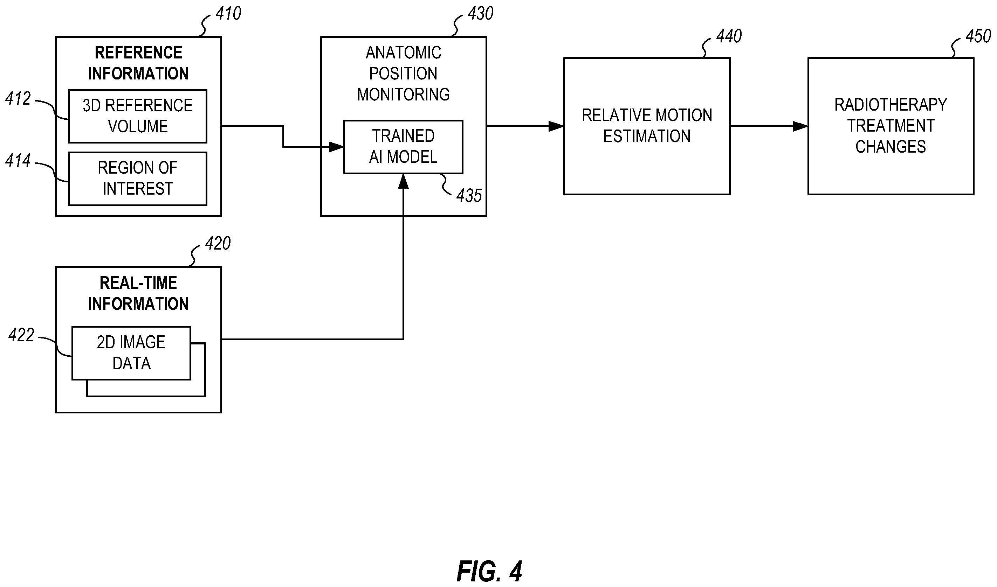

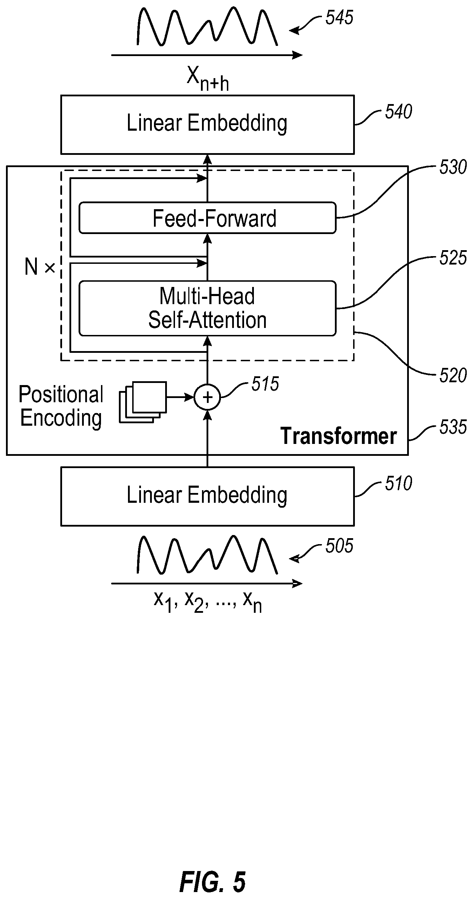

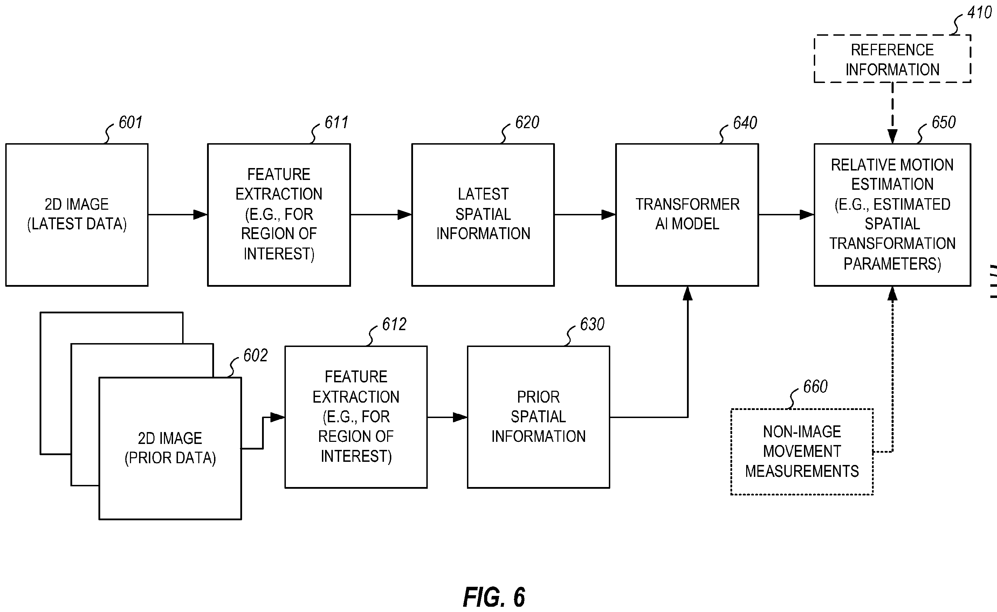

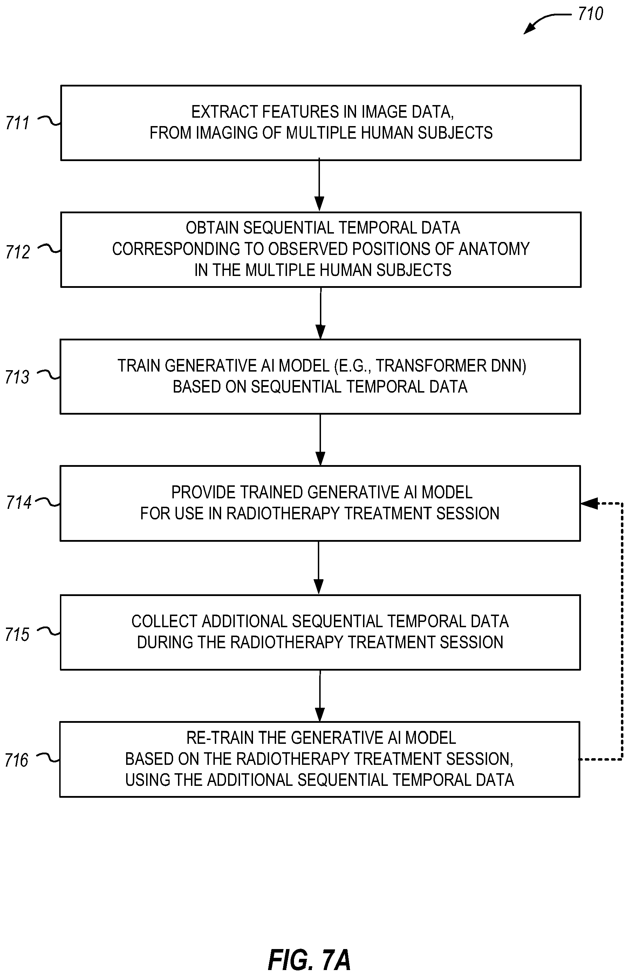

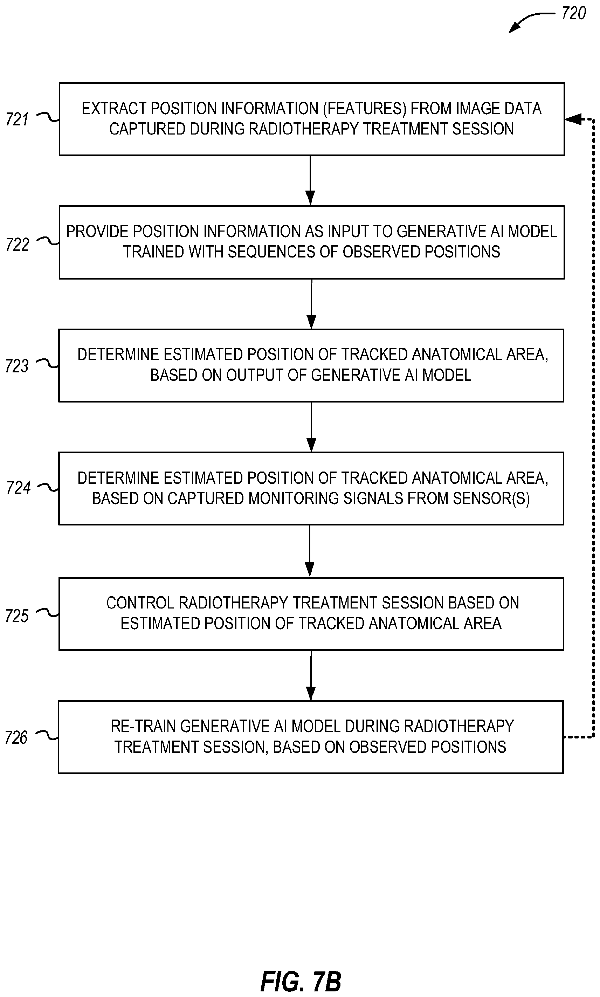

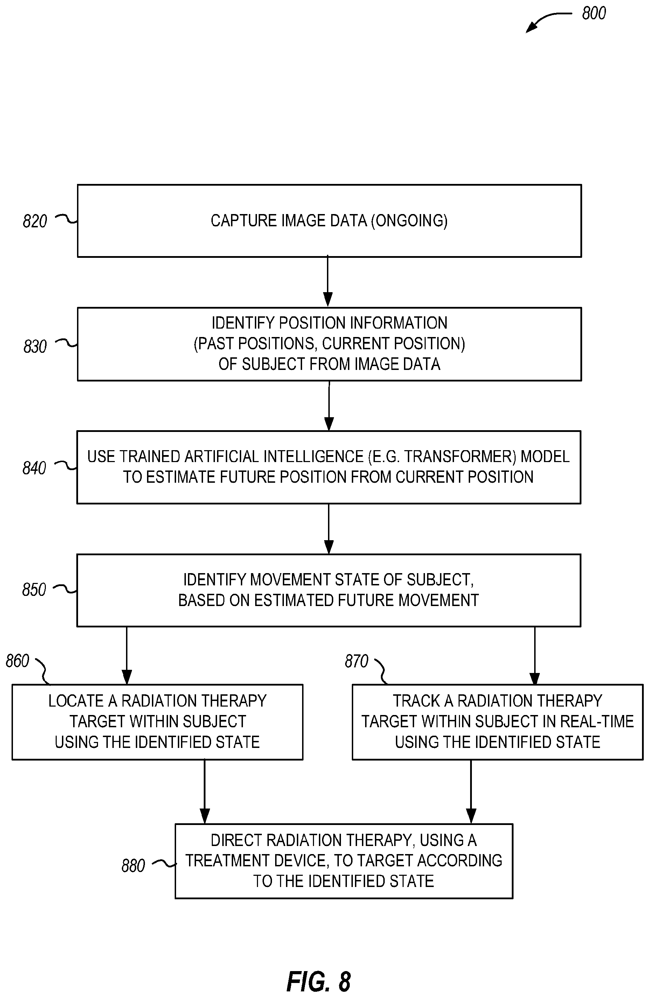

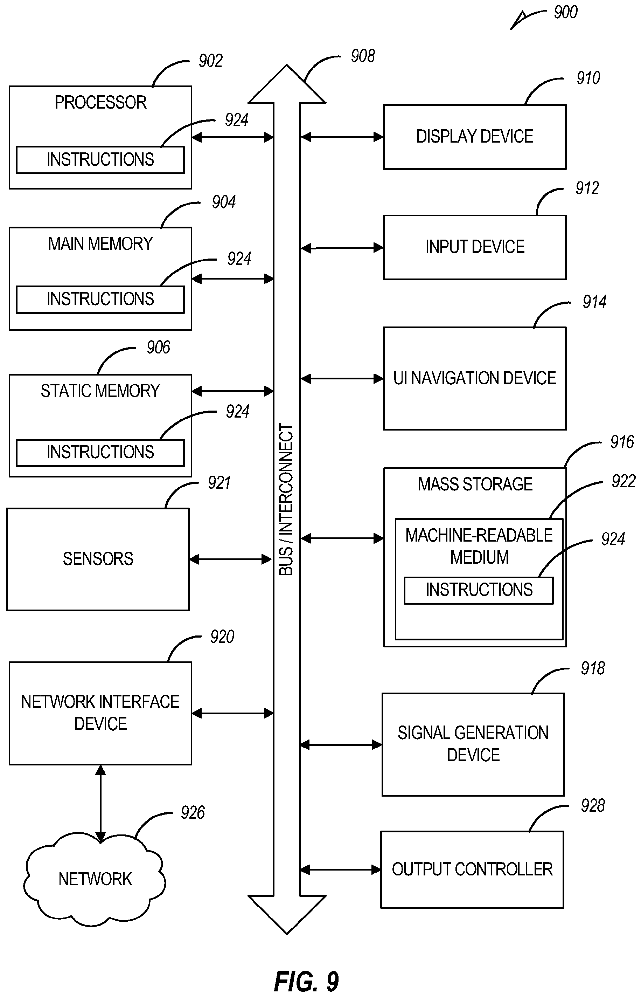

The following paragraphs provide an overview of example radiotherapy system implementations and treatment use cases (with reference to **FIGS. 2A, 2B,** and 3), including with the use of computing systems and hardware implementations (with reference to **FIGS. 1** and **9).** The following then continues with a discussion of a workflow to perform APM (with reference to **FIGS. 4** and **6),** and specific usage of a Transformer model (with reference to **FIG. 5).** Finally, a discussion of machine learning techniques is provided, along with further processing details of training and using a machine learning model, including training and use in a radiotherapy therapy session for a particular patient (with reference to **FIGS. 7A, 7B,** and **8).**

**FIG. 1** illustrates a radiotherapy system 100 adapted for using machine learning models for assisting anatomic position monitoring. The anatomic position monitoring may be used to determine a patient state to enable the radiotherapy system 100 to provide radiation therapy to a patient based on specific aspects of captured medical imaging data. The radiotherapy system includes an image processing computing system 110 which hosts patient state processing logic 120. The image processing computing system 110 may be connected to a network (not shown), and such network may be connected to the Internet. For instance, a network can connect the image processing computing system 110 with one or more medical information sources (e.g., a radiology information system (RIS), a medical record system (e.g., an electronic medical record (EMR) / electronic health record (EHR) system), an oncology information system (OIS)), one or more image data sources 150, an image acquisition device 170, and a treatment device 180 (e.g., a radiation therapy device). As an example, the image processing computing system 110 can be configured to perform image patient state operations by executing instructions or data from the patient state processing logic 120, as part of operations to generate and customize radiation therapy treatment plans to be used by the treatment device 180.

The image processing computing system 110 may include processing circuitry 112, memory 114, a storage device 116, and other hardware and software-operable features such as a user interface 140, communication interface, and the like. The storage device 116 may store computer-executable instructions, such as an operating system, radiation therapy treatment plans (e.g., original treatment plans, adapted treatment plans, or the like), software programs (e.g., radiotherapy treatment plan software, artificial intelligence implementations such as machine learning models, deep learning models, and neural networks, etc.), and any other computer-executable instructions to be executed by the processing circuitry 112.

In an example, the processing circuitry 112 may include a processing device, such as one or more general-purpose processing devices such as a microprocessor, a central processing unit (CPU), a graphics processing unit (GPU), an accelerated processing unit (APU), or the like. More particularly, the processing circuitry 112 may be a complex instruction set computing (CISC) microprocessor, a reduced instruction set computing (RISC) microprocessor, a very long instruction Word (VLIW) microprocessor, a processor implementing other instruction sets, or processors implementing a combination of instruction sets. The processing circuitry 112 may also be implemented by one or more special-purpose processing devices such as an application specific integrated circuit (ASIC), a field programmable gate array (FPGA), a digital signal processor (DSP), a System on a Chip (SoC), or the like. As would be appreciated by those skilled in the art, in some examples, the processing circuitry 112 may be a special-purpose processor, rather than a general-purpose processor. The processing circuitry 112 may include one or more known processing devices, such as a microprocessor from the Pentium™, Core™, Xeon™, or Itanium® family manufactured by Intel™, the Turion™, Athlon™, Sempron™, Opteron™, FX™, Phenom™ family manufactured by AMD™, or any of various processors manufactured by Sun Microsystems. The processing circuitry 112 may also include graphical processing units such as a GPU from the GeForce®, Quadro®, Tesla® family manufactured by Nvidia™, GMA, Iris™ family manufactured by Intel™, or the Radeon™ family manufactured by AMD™. The processing circuitry 112 may also include accelerated processing units such as the Xeon Phi™ family manufactured by Intel™. The disclosed embodiments are not limited to any type of processor(s) otherwise configured to meet the computing demands of identifying, analyzing, maintaining, generating, and/or providing large amounts of data or manipulating such data to perform the methods disclosed herein. In addition, the term "processor" may include more than one processor, for example, a multi-core design or a plurality of processors each having a multi-core design. The processing circuitry 112 can execute sequences of computer program instructions, stored in memory 114, and accessed from the storage device 116, to perform various operations, processes, methods that will be explained in greater detail below.

The memory 114 may comprise read-only memory (ROM), a phase-change random access memory (PRAM), a static random access memory (SRAM), a flash memory, a random access memory (RAM), a dynamic random access memory (DRAM) such as synchronous DRAM (SDRAM), an electrically erasable programmable read-only memory (EEPROM), a static memory (e.g., flash memory, flash disk, static random access memory) as well as other types of random access memories, a cache, a register, a compact disc read-only memory (CD-ROM), a digital versatile disc (DVD) or other optical storage, a cassette tape, other magnetic storage device, or any other non-transitory medium that may be used to store information including image, data, or computer executable instructions (e.g., stored in any format) capable of being accessed by the processing circuitry 112, or any other type of computer device. For instance, the computer program instructions can be accessed by the processing circuitry 112, read from the ROM, or any other suitable memory location, and loaded into the RAM for execution by the processing circuitry 112.

The storage device 116 may constitute a drive unit that includes a machine-readable medium on which is stored one or more sets of instructions and data structures (e.g., software) embodying or utilized by any one or more of the methodologies or functions described herein (including, in various examples, the patient state processing logic 120 and the user interface 140). The instructions may also reside, completely or at least partially, within the memory 114 and/or within the processing circuitry 112 during execution thereof by the image processing computing system 110, with the memory 114 and the processing circuitry 112 also constituting machine-readable media.

The memory 114 or the storage device 116 may constitute a non-transitory computer-readable medium. For example, the memory 114 or the storage device 116 may store or load instructions for one or more software applications on the computer-readable medium. Software applications stored or loaded with the memory 114 or the storage device 116 may include, for example, an operating system for common computer systems as well as for software-controlled devices. The image processing computing system 110 may also operate a variety of software programs comprising software code for implementing the patient state processing logic 120 and the user interface 140. Further, the memory 114 and the storage device 116 may store or load an entire software application, part of a software application, or code or data that is associated with a software application, which is executable by the processing circuitry 112. In a further example, the memory 114 or the storage device 116 may store, load, or manipulate one or more radiation therapy treatment plans, imaging data, patient state data, dictionary entries, artificial intelligence model data, labels, and mapping data, etc. It is contemplated that software programs may be stored not only on the storage device 116 and the memory 114 but also on a removable computer medium, such as a hard drive, a computer disk, a CD-ROM, a DVD, a HD-DVD, a Blu-Ray DVD, USB flash drive, a SD card, a memory stick, or any other suitable medium; such software programs may also be communicated or received over a network.

Although not depicted, the image processing computing system 110 may include a communication interface, network interface card, and communications circuitry. An example communication interface may include, for example, a network adaptor, a cable connector, a serial connector, a USB connector, a parallel connector, a high-speed data transmission adaptor (e.g., such as fiber optic, USB 3.0, thunderbolt, and the like), a wireless network adaptor (e.g., such as a IEEE 802.11/Wi-Fi adapter), a telecommunication adapter (e.g., to communicate with 3G, 4G/LTE, and 5G networks and the like), and similar networking components. Such a communication interface may include one or more digital and/or analog communication devices that permit a machine to communicate with other machines and devices, such as remotely located components, via a network. The network may provide the functionality of a local area network (LAN), a wireless network, a cloud computing environment (e.g., software as a service, platform as a service, infrastructure as a service, etc.), a client-server, a wide area network (WAN), and the like. For example, network may be a LAN or a WAN that may include other systems (including additional image processing computing systems or image-based components associated with medical imaging or radiotherapy operations).

In an example, the image processing computing system 110 may obtain image data 160 from the image data source 150, for hosting on the storage device 116 and the memory 114. In an example, the software programs operating on the image processing computing system 110 may convert or transform medical images of one format (e.g., MRI) to another format (e.g., CT), such as by producing synthetic images, such as a pseudo-CT image. In another example, the software programs may register or associate a patient medical image (e.g., a CT image or an MR image) with that patient's dose distribution of radiotherapy treatment (e.g., also represented as an image) so that corresponding image voxels and dose voxels are appropriately associated. In another example, the software programs may visualize, hide, emphasize, or de-emphasize some aspect of anatomical features, patient measurements, patient state information, or dose or treatment information, within medical images. The storage device 116 and memory 114 may store and host data to perform these purposes, including the image data 160, patient data, and other data required to create and implement a radiation therapy treatment plan and associated patient state estimation operations.

The processing circuitry 112 may be communicatively coupled to the memory 114 and the storage device 116, and the processing circuitry 112 may be configured to execute computer executable instructions stored thereon from either the memory 114 or the storage device 116. The processing circuitry 112 may execute instructions to cause medical images from the image data 160 to be received or obtained in memory 114, and processed using the patient state processing logic 120. For example, the image processing computing system 110 may receive image data 160 from the image acquisition device 170 or image data sources 150 via a communication interface and network to be stored or cached in the storage device 116. The processing circuitry 112 may also send or update medical images stored in memory 114 or the storage device 116 via a communication interface to another database or data store (e.g., a medical facility database). In some examples, one or more of the systems may form a distributed computing/simulation environment that uses a network to collaboratively perform the embodiments described herein (such as in an edge computing environment). In addition, such network may be connected to the Internet to communicate with servers and clients that reside remotely on the Internet.

In further examples, the processing circuitry 112 may utilize software programs (e.g., a treatment planning software) along with the image data 160 and other patient data to create a radiation therapy treatment plan. In an example, the image data 160 may include 2D or 3D volume imaging, such as from a CT or MR. In addition, the processing circuitry 112 may utilize aspects of AI such as machine learning, deep learning, and neural networks to generate or control various aspects of the treatment plan, including in response to an estimated patient state or patient movement as discussed in the following examples.

For instance, such software programs may utilize patient state processing logic 120 to implement a patient state determination workflow 130, using the techniques and Transformer AI models further discussed herein. The processing circuitry 112 may subsequently then modify and transmit the executable radiation therapy treatment plan via a communication interface and the network to the treatment device 180, where the radiation therapy plan will be used to treat a patient with radiation via the treatment device, consistent with results of the patient state determination workflow 130. Other outputs and uses of the software programs and the patient state determination workflow 130 may occur with use of the image processing computing system 110. As discussed further below, the processing circuitry 112 may execute a software program that invokes the patient state processing logic 120 to implement functions including aspects of image processing and registration, feature extraction, machine learning model processing, and the like.

In an example, the image data 160 may include one or more MRI images (e.g., 2D MRI, 3D MRI, 2D streaming MRI, 4D MRI, 4D volumetric MRI, 4D cine MRI, etc.), functional MRI images (e.g., fMRI, DCE-MRI, diffusion MRI), Computed Tomography (CT) images (e.g., 2D CT, Cone beam CT, 3D CT, 4D CT), ultrasound images (e.g., 2D ultrasound, 3D ultrasound, 4D ultrasound), Positron Emission Tomography (PET) images, X-ray images, fluoroscopic images, radiotherapy portal images, Single-Photo Emission Computed Tomography (SPECT) images, computer generated synthetic images (e.g., pseudo-CT images) and the like. Further, the image data 160 may also include or be associated with auxiliary information, such as segmentations/contoured images, or dose images. In an example, the image data 160 may be received from the image acquisition device 170 and stored in one or more of the image data sources 150 (e.g., a Picture Archiving and Communication System (PACS), a Vendor Neutral Archive (VNA), a medical record or information system, a data warehouse, etc.). Accordingly, the image acquisition device 170 may comprise a MRI imaging device, a CT imaging device, a PET imaging device, an ultrasound imaging device, a fluoroscopic device, a SPECT imaging device, an integrated Linear Accelerator and MRI imaging device, or other medical imaging devices for obtaining the medical images of the patient. The image data 160 may be received and stored in any type of data or any type of format (e.g., in a Digital Imaging and Communications in Medicine (DICOM) format) that the image acquisition device 170 and the image processing computing system 110 may use to perform operations consistent with the disclosed embodiments.

In an example, the image acquisition device 170 may be integrated with the treatment device 180 as a single apparatus (e.g., an MRI device combined with a linear accelerator, also referred to as an "MR-LINAC", as shown and described in FIG. 3 below). Such an MR-LINAC can be used, for example, to precisely determine a location of a target organ or a target tumor in the patient, so as to direct radiation therapy accurately according to the radiation therapy treatment plan to a predetermined target. For instance, a radiation therapy treatment plan may provide information about a particular radiation dose to be applied to each patient. The radiation therapy treatment plan may also include other radiotherapy information, such as beam angles, dose-histogram-volume information, the number of radiation beams to be used during therapy, the dose per beam, and the like.

The image processing computing system 110 may communicate with an external database through a network to send/receive a plurality of various types of data related to image processing and radiotherapy operations. For example, an external database may include machine data that is information associated with the treatment device 180, the image acquisition device 170, or other machines relevant to radiotherapy or medical procedures. Machine data information may include radiation beam size, arc placement, beam on and off time duration, machine parameters, segments, multi-leaf collimator (MLC) configuration, gantry speed, MRI pulse sequence, and the like. The external database may be a storage device and may be equipped with appropriate database administration software programs. Further, such databases or data sources may include a plurality of devices or systems located either in a central or a distributed manner.

The image processing computing system 110 can collect and obtain data, and communicate with other systems, via a network using one or more communication interfaces, which are communicatively coupled to the processing circuitry 112 and the memory 114. For instance, a communication interface may provide communication connections between the image processing computing system 110 and radiotherapy system components (e.g., permitting the exchange of data with external devices). For instance, the communication interface may in some examples have appropriate interfacing circuitry from an output device 142 or an input device 144 to connect to the user interface 140, which may be a hardware keyboard, a keypad, or a touch screen through which a user may input information into the radiotherapy system 100.

As an example, the output device 142 may include a display device which outputs a representation of the user interface 140 and one or more aspects, visualizations, or representations of the medical images. The output device 142 may include one or more display screens that display medical images, interface information, treatment planning parameters (e.g., contours, dosages, beam angles, labels, maps, etc.) treatment plans, a target, localizing a target or tracking a target, patient state estimations (e.g., a 3D volume), or any related information to the user. The input device 144 connected to the user interface 140 may be a keyboard, a keypad, a touch screen or any type of device that a user may input information to the radiotherapy system 100. Alternatively, the output device 142, the input device 144, and features of the user interface 140 may be integrated into a single device such as a smartphone or tablet computer, e.g., Apple iPad®, Lenovo Thinkpad®, Samsung Galaxy®, etc.

Furthermore, many components of the radiotherapy system 100 may be implemented with a virtual machine (e.g., via VMWare, Hyper-V, and the like virtualization platforms). For instance, a virtual machine can be software that functions as hardware. Therefore, a virtual machine can include at least one or more virtual processors, one or more virtual memories, and one or more virtual communication interfaces that together function as hardware. For example, the image processing computing system 110, the image data sources 150, or like components, may be implemented as a virtual machine or within a cloud-based virtualization environment.

The patient state processing logic 120 or other software programs may cause the computing system to communicate with the image data sources 150 to read images into memory 114 and the storage device 116, or store images or associated data from the memory 114 or the storage device 116 to and from the image data sources 150. For example, the image data source 150 may be configured to store and provide a plurality of images (e.g., 3D MRI, 4D MRI, 2D MRI slice images, CT images, 2D Fluoroscopy images, X-ray images, raw data from MR scans or CT scans, Digital Imaging and Communications in Medicine (DICOM) metadata, etc.) that the image data source 150 hosts, from image sets in image data 160 obtained from one or more patients via the image acquisition device 170, including in real-time settings, defined further below. The image data source 150 or other databases may also store data to be used by the patient state processing logic 120 when executing a software program that performs patient state estimation operations, or when creating, monitoring, or modifying radiation therapy treatment plans. Further, various databases may store machine learning or other AI models, including the algorithm parameters, weights, or other data constituting the model learned by the network and the resulting predicted or estimated data. The image processing computing system 110 thus may obtain and/or receive the image data 160 (e.g., 2D MRI slice images, CT images, 2D Fluoroscopy images, X-ray images, 3D MRI images, 4D MRI images, etc.) from the image data source 150, the image acquisition device 170, the treatment device 180 (e.g., a MR-LINAC), or other information systems, in connection with performing image patient state estimation as part of treatment or diagnostic operations.

The image acquisition device 170 can be configured to acquire one or more images of the patient's anatomy relevant to a region of interest (e.g., a target organ, a target tumor or both). Each image, typically a 2D image or slice, can include one or more parameters (e.g., a 2D slice thickness, an orientation, an origin and field of view, etc.). In an example, the image acquisition device 170 can acquire a 2D slice in any orientation. For example, an orientation of the 2D slice can include a sagittal orientation, a coronal orientation, or an axial orientation. The processing circuitry 112 can adjust one or more parameters, such as the thickness and/or orientation of the 2D slice, to include the target organ and/or target tumor. In an example, 2D slices can be determined from information such as a 3D MRI volume. Such 2D slices can be acquired by the image acquisition device 170 in "real-time" while a patient is undergoing radiation therapy treatment, for example, when using the treatment device 180 (with "real-time" meaning, in an example, acquiring the data in 10 milliseconds or less). In another example for some applications, real-time may include a timeframe within (e.g., up to) 300 milliseconds. In an example, real-time may include a time period fast enough for a clinical problem being solved by techniques described herein. In this example, real-time may vary depending on target speed, radiotherapy margins, lag, response time of a treatment device, etc.

The patient state processing logic 120 in the image processing computing system 110 is depicted as implementing a patient state determination workflow 130 with various aspects of monitoring and estimation of a patient state provided by models or algorithms. In an example, the patient state determination workflow 130 uses a real-time image input stream 132 (e.g., 2D partial measurements, such as from a CT or MR), which is analyzed by anatomic position monitoring 136 functions to estimate a patient state. In a further example, the patient state determination workflow 130 uses a real-time sensor data stream 134 (e.g., breathing belt measurements, other external, non-image sensor measurements) which is analyzed by anatomic position monitoring 136 functions to estimate or refine the patient state.

The patient state determination workflow 130 further involves aspects of anatomic position monitoring 136, such as determined within the trained AI model (e.g., Transformer deep learning model) discussed in further examples below. The data provided from anatomic position monitoring 136 may be used for producing or controlling a patient state estimation 138. The patient state estimation 138 may produce data that is used to control the treatment device 180 or other aspects of the radiotherapy session.

FIG. 2A illustrates a radiation therapy device 202 that may include a radiation source, such as an X-ray source or a linear accelerator, a couch 216, an imaging detector 214, and a radiation therapy output 204. The radiation therapy device 202 may be configured to emit a radiation beam 208 to provide therapy to a patient. The radiation therapy output 204 can include one or more attenuators or collimators, such as an MLC. A MLC may be used for shaping, directing, or modulating an intensity of a radiation therapy beam to the specified target locus within the patient. The leaves of the MLC, for instance, can be automatically positioned to define an aperture approximating a tumor cross-section or projection, and cause modulation of the radiation therapy beam. For example, the leaves can include metallic plates, such as comprising tungsten, with a long axis of the plates oriented parallel to a beam direction and having ends oriented orthogonally to the beam direction. Further, a "state" of the MLC can be adjusted adaptively during a course of radiation therapy treatment, such as to establish a therapy beam that better approximates a shape or location of the tumor or other target locus.

Referring back to FIG. 2A, a patient can be positioned in a region 212 and supported by the treatment couch 216 to receive a radiation therapy dose, according to a radiation therapy treatment plan. The radiation therapy output 204 can be mounted or attached to a gantry 206 or other mechanical support. One or more chassis motors (not shown) may rotate the gantry 206 and the radiation therapy output 204 around couch 216 when the couch 216 is inserted into the treatment area. In an example, gantry 206 may be continuously rotatable around couch 216 when the couch 216 is inserted into the treatment area. In another example, gantry 206 may rotate to a predetermined position when the couch 216 is inserted into the treatment area. For example, the gantry 206 can be configured to rotate the therapy output 204 around an axis ("A"). Both the couch 216 and the radiation therapy output 204 can be independently moveable to other positions around the patient, such as moveable in transverse direction ("T"), moveable in a lateral direction ("L"), or as rotation about one or more other axes, such as rotation about a transverse axis (indicated as "R"). A controller communicatively connected to one or more actuators (not shown) may control the couch 216 movements or rotations in order to properly position the patient in or out of the radiation beam 208 according to a radiation therapy treatment plan. Both the couch 216 and the gantry 206 are independently moveable from one another in multiple degrees of freedom, which allows the patient to be positioned such that the radiation beam 208 can target the tumor precisely. The MLC may be integrated and included within gantry 206 to deliver the radiation beam 208 of a certain shape.

The coordinate system (including axes A, T, and L) shown in FIG. 2A can have an origin located at an isocenter 210. The isocenter can be defined as a location where the central axis of the radiation beam 208 intersects the origin of a coordinate axis, such as to deliver a prescribed radiation dose to a location on or within a patient. Alternatively, the isocenter 210 can be defined as a location where the central axis of the radiation beam 208 intersects the patient for various rotational positions of the radiation therapy output 204 as positioned by the gantry 206 around the axis A. As discussed herein, the gantry angle corresponds to the position of gantry 206 relative to axis A, although any other axis or combination of axes can be referenced and used to determine the gantry angle.

Gantry 206 may also have an attached imaging detector 214. The imaging detector 214 is preferably located opposite to the radiation source, and in an example, the imaging detector 214 can be located within a field of the radiation beam 208.

The imaging detector 214 can be mounted on the gantry 206 (preferably opposite the radiation therapy output 204), such as to maintain alignment with the radiation beam 208. The imaging detector 214 rotates about the rotational axis as the gantry 206 rotates. In an example, the imaging detector 214 can be a flat panel detector (e.g., a direct detector or a scintillator detector). In this manner, the imaging detector 214 can be used to monitor the radiation beam 208 or the imaging detector 214 can be used for imaging the patient's anatomy, such as portal imaging. The control circuitry of the radiation therapy device 202 may be integrated within the radiotherapy system 100 or remote from it.

In an illustrative example, one or more of the couch 216, the therapy output 204, or the gantry 206 can be automatically positioned, and the therapy output 204 can establish the radiation beam 208 according to a specified dose for a particular therapy delivery instance. A sequence of therapy deliveries can be specified according to a radiation therapy treatment plan, such as using one or more different orientations or locations of the gantry 206, couch 216, or therapy output 204. The therapy deliveries can occur sequentially, but can intersect in a desired therapy locus on or within the patient, such as at the isocenter 210. A prescribed cumulative dose of radiation therapy can thereby be delivered to the therapy locus while damage to tissue near the therapy locus can be reduced or avoided.

FIG. 2B illustrates a radiation therapy device 202 that may include a combined LINAC and an imaging system, such as a CT imaging system. The radiation therapy device 202 can include an MLC (not shown). The CT imaging system can include an imaging X-ray source 218, such as providing X-ray energy in a kiloelectron-Volt (keV) energy range. The imaging X-ray source 218 can provide a fan-shaped and/or a conical radiation beam 208 directed to an imaging detector 222, such as a flat panel detector. The radiation therapy device 202 can be similar to the system described in relation to FIG. 2A, such as including a radiation therapy output 204, a gantry 206, a couch 216, and another imaging detector 214 (such as a flat panel detector). The X-ray source 218 can provide a comparatively-lower-energy X-ray diagnostic beam, for imaging.

In the illustrative example of FIG. 2B, the radiation therapy output 204 and the X-ray source 218 can be mounted on the same rotating gantry 206, rotationally separated from each other by 90 degrees. In another example, two or more X-ray sources can be mounted along the circumference of the gantry 206, such as each having its own detector arrangement to provide multiple angles of diagnostic imaging concurrently. Similarly, multiple radiation therapy outputs 204 can be provided.

FIG. 3 depicts a radiation therapy system 300 that can include combining a radiation therapy device 202 and an imaging system, such as a magnetic resonance (MR) imaging system (e.g., known in the art as an MR-LINAC) consistent with the disclosed examples. As shown, system 300 may include a couch 216, an image acquisition device 320, and a radiation delivery device 330. System 300 delivers radiation therapy to a patient in accordance with a radiotherapy treatment plan. In some examples, image acquisition device 320 may correspond to image acquisition device 170 in FIG. 1 that may acquire origin images of a first modality (e.g., an MRI image) or destination images of a second modality (e.g., an CT image).

Couch 216 may support a patient (not shown) during a treatment session. In some implementations, couch 216 may move along a horizontal translation axis (labelled "I"), such that couch 216 can move the patient resting on couch 216 into and/or out of system 300. Couch 216 may also rotate around a central vertical axis of rotation, transverse to the translation axis. To allow such movement or rotation, couch 216 may have motors (not shown) enabling the couch 216 to move in various directions and to rotate along various axes. A controller (not shown) may control these movements or rotations in order to properly position the patient according to a treatment plan.

In some examples, image acquisition device 320 may include an MRI machine used to acquire 2D or 3D MRI images of the patient before, during, and/or after a treatment session. Image acquisition device 320 may include a magnet 321 for generating a primary magnetic field for magnetic resonance imaging. The magnetic field lines generated by operation of magnet 321 may run substantially parallel to the central translation axis I. Magnet 321 may include one or more coils with an axis that runs parallel to the translation axis I. In some examples, the one or more coils in magnet 321 may be spaced such that a central window 323 of magnet 321 is free of coils. In other examples, the coils in magnet 321 may be thin enough or of a reduced density such that they are substantially transparent to radiation of the wavelength generated by radiation delivery device 330. Image acquisition device 320 may also include one or more shielding coils, which may generate a magnetic field outside magnet 321 of approximately equal magnitude and opposite polarity in order to cancel or reduce any magnetic field outside of magnet 321. As described below, radiation source 331 of radiation delivery device 330 may be positioned in the region where the magnetic field is cancelled, at least to a first order, or reduced.

Image acquisition device 320 may also include two gradient coils 325 and 326, which may generate a gradient magnetic field that is superposed on the primary magnetic field. Coils 325 and 326 may generate a gradient in the resultant magnetic field that allows spatial encoding of the protons so that their position can be determined. Gradient coils 325 and 326 may be positioned around a common central axis with the magnet 321 and may be displaced along that central axis. The displacement may create a gap, or window, between coils 325 and 326. In examples where magnet 321 can also include a central window 323 between coils, the two windows may be aligned with each other.

In some examples, image acquisition device 320 may be an imaging device other than an MRI, such as an X-ray, a CT, a CBCT, a spiral CT, a PET, a SPECT, an optical tomography, a fluorescence imaging, ultrasound imaging, radiotherapy portal imaging device, or the like. As would be recognized by one of ordinary skill in the art, the above description of image acquisition device 320 concerns certain examples and is not intended to be limiting.

Radiation delivery device 330 may include the radiation source 331, such as an X-ray source or a LINAC, and an MLC 332. Radiation delivery device 330 may be mounted on a chassis 335. One or more chassis motors (not shown) may rotate the chassis 335 around the couch 216 when the couch 216 is inserted into the treatment area. In an example, the chassis 335 may be continuously rotatable around the couch 216, when the couch 216 is inserted into the treatment area.

Chassis 335 may also have an attached radiation detector (not shown), preferably located opposite to radiation source 331 and with the rotational axis of the chassis 335 positioned between the radiation source 331 and the detector. Further, the radiation delivery device 330 may include control circuitry (not shown) used to control, for example, one or more of the couch 216, image acquisition device 320, and radiation delivery device 330. The control circuitry of the radiation delivery device 330 may be integrated within the system 300 or remote from it.

During a radiotherapy treatment session, a patient may be positioned on couch 216. System 300 may then move couch 216 into the treatment area defined by the magnet 321, coils 325, 326, and chassis 335. Control circuitry may then control radiation source 331, MLC 332, and the chassis motor(s) to deliver radiation to the patient through the window between coils 325 and 326 according to a radiotherapy treatment plan.

**FIG. 2A, FIG. 2B,** and **FIG. 3** generally illustrate examples of a radiation therapy device configured to provide radiotherapy treatment to a patient, including a configuration where a radiation therapy output can be rotated around a central axis (e.g., an axis "A"). Other radiation therapy output configurations can be used. For example, a radiation therapy output can be mounted to a robotic arm or manipulator having multiple degrees of freedom. In yet another example, the therapy output can be fixed, such as located in a region laterally separated from the patient, and a platform supporting the patient can be used to align a radiation therapy isocenter with a specified target locus within the patient.

As noted above, when performing radiation therapy, underlying 3D patient motion must be estimated and tracked in order to accurately deliver radiation therapy treatment at a correct location. To do this, radiotherapy treatment techniques involve an estimation of the relative motion of a specific object contained in a specified region of interest, relative to a reference volume which contains auxiliary information such as contoured regions of interest or the dose plan. This estimation and monitoring of a location for a specific object is referred to herein as anatomic position monitoring (APM).

FIG. 4 provides a high-level view of APM operations. The goal of APM is to produce a real-time relative motion estimation 440 of an object contained in a region of interest, relative to its position in a known 3D reference space. The relative motion estimation 440 then can be used to adjust the radiotherapy treatment and cause radiotherapy treatment changes 450 that are directed to one or more regions of interest within the 3D reference space. It will be understood that a variety of techniques for adjusting or modifying the location, type, amount, or characteristics of radiotherapy treatment based on motion may be utilized, based upon the identification of the anatomic position and an estimate of relative motion.

The operations in FIG. 4, in more detail, illustrate how reference information 410 for a human subject may be correlated to movement changes that are identified from real-time information 420 for the human subject. The reference information 410 may include imaging data from a 3D reference volume 412 (e.g., produced from an MRI or CT scan), and a definition of a region of interest 414 (e.g., a mask or area defining a target organ, a target tumor or both). The real-time information 420 may include 2D imaging data 422 (e.g., produced from 2D MR images or from kV projection imaging (x-ray images)), collected over time from a single or multiple orientations (e.g., a first image captured at a coronal plane, and a second image captured at a sagittal plane). Other forms of real-time information 420, not depicted, may include position monitoring signals (e.g., a signal from a breathing belt, sensor data, etc.) captured from observed patient body movement.

Based on input data of the 3D reference volume 412, an accompanying tracking of the region of interest 414, and real-time information 420 (e.g., instantaneous, ongoing) relating to the patient (e.g., 2D imaging data 422 captured on an ongoing basis), an APM model 430 analyzes the real-time information 420 to determine movement relative to the reference information 410.

The APM model 430 may include one or more algorithms, such as a trained AI model 435 that comprises a trained Transformer deep learning model or other artificial intelligence algorithm implementation. The APM model 430 is trained to estimate motion in a 3D space based on analysis of the real-time information 410, with such real-time information potentially including the most recent image as well as other images captured over a period of time. It will be understood that the APM model 430 may also integrate or use other types of image and data processing techniques, including those which do not implement AI models or AI functionality.

The APM model 430 estimates a motion of one or more anatomical areas (relative to a reference volume) from 2D images and/or other data of the patient. The APM model 430 uses the trained AI model 435 (e.g., a Transformer model) to then predict an estimate of future motion from observations of previous motion. Thus, the "trained" AI model is a generative model that is specially trained to predict or estimate future position, motion, or some derivative of these actions.

In an example, the APM model 430 uses the trained AI model 435 to generate and output a relative motion estimation 440 in the form of transformation parameters that describe the motion of the tracked region relative to the reference volume 412. The relative motion estimation 440 may be processed to produce radiotherapy treatment changes 450 that dynamically gate the radiotherapy beam (e.g., turn the beam on or off in real-time), or dynamically effect a change in direction, shape, position, intensity, amount, or type of a beam in the radiotherapy treatment. As a simple example, radiotherapy treatment changes 450 may include control of a radiotherapy beam, such as starting or stopping radiotherapy treatment output, or turning a radiotherapy beam on or off, based on movement caused by patient breathing.

The following paragraphs provide an overview of the use of temporal prediction in the APM model 430 or a similar APM processing workflow, based on the usage of a Transformer deep learning model as the trained AI model 435. It will be understood that this general framework for temporal prediction in APM may be adapted for the use of other AI models and variations. Further, it will be understood that the following deployment of the Transformer may include a Transformer that is trained on data specific to the patient (subject being treated), specific to a larger population of patients (other patients), or both. For example, a model may be trained on a population of data and then fine-tuned (trained) with patient data to achieve better performance for each particular patient.

To more precisely represent motion estimation, consider the following equation. Let $\vec{p}_t \in R^3$ be a 3D vector describing the estimated target position at time index t. The goal of prediction is to find a mapping $f: \vec{x} \to \vec{y}$ to map input features $\vec{x}$ describing the current position (and, commonly, several of the most recent previous positions) to the future position $\vec{y}$. During treatment, at time t, a training pair can be formed:

$$(\vec{x}, \vec{y}) = \left( \left\{ \vec{p}_{t-h-n_{input}+1}, \dots, \vec{p}_{t-h} \right\}, \vec{p}_t \right)$$

**(EQUATION 1)**

where $h$ is the prediction horizon and $n_{input}$ is the total number of samples in the input vector $\vec{x}$. A moving-window strategy is used to collect training pairs throughout treatment. These samples are placed in a circular buffer with a fixed maximum length $n_{train}^{max}$. In other words, training samples are placed into the buffer until $n_{train}^{max}$ samples are held; afterwards, for each new training sample, it is placed in the buffer, over-writing the oldest training sample.

The prediction model (e.g., the Transformer predictive model) is updated periodically by re-training throughout treatment at intervals $\Delta_{update}$. This technique allows the prediction model to adapt to slow changes (e.g., drift) in respiratory motion. Furthermore, $\Delta_{update}$ is constrained to be greater than the time required to train the particular model $f: \vec{x} \to \vec{y}$. This design choice allows model training to be performed on a single computer thread throughout treatment, simplifying implementation. Given a model trained at time t*, the model therefore becomes available for prediction at time t $\geq$ t* + $\Delta_{update}$.

This framework allows the use of a Transformer deep learning model to be adapted in APM operations. As will be understood, the building block of the Transformer model is the scaled dot-product attention (or "self-attention") mechanism. First, the input features $\vec{x}$ are linearly projected to a high-dimensional space $h_t \in R^d$, extracting $d$ features. Positional encodings, which provide additional information about the relative temporal position of each value in the sequence, may be added to the high-dimensional features,. Then, the high-dimensional feature vectors are linearly projected to a set of queries $Q \in R^{n_q \times d_q}$, keys $K \in R^{n_k \times d_k}$ and values $V \in R^{n_v \times d_v}$. The vector dimensionality $d_q$ equals to $d_k$, the number of keys $n_k$ equals to the number of values $n_v$. The output of the attention layer is given by computing the weighted sum of the values, where the attention scores $S \in R^{n_q \times n_k}$ are calculated from the queries and keys as follows:

$$A(Q, K, V) = softmax(S) = softmax\left( \frac{QK^T}{\sqrt{d_k}} \right) V$$

**(EQUATION 2)**

where the softmax function is used to normalize the scaled dot-product attention scores S. This scaled dot-product attention layer may, in some embodiments, be extended to so-called "Multi-head attention", which creates different representation subspaces for the attention:

$$Multihead(Q, K, V) = Concat(head_1, head_2, \dots, head_N)W^O$$

**(EQUATION 3)**

where $head_i = A\left( QW_i^Q, KW_i^K, VW_i^V \right)$. In each attention head, the inputs $Q, K, V$ are firstly linearly projected by weights $W_i^Q \in R^{d_{model} \times d_q}$, $W_i^K \in R^{d_{model} \times d_k}$ and $W_i^V \in R^{d_{model} \times d_v}$ respectively, where $d_{model}$ is a model hyperparameter. The outputs of each head are combined via concatenation and then finally linearly projected.

**FIG. 5** illustrates an example of an attention-based Transformer model 535 adapted for target position forecasting and temporal prediction in APM. The Transformer model 535 may be structured based on a "decoder-only" Transformer architecture, similar to that of the Generative Pre-trained Transformer 3 (GPT-3) model used for natural language processing.

In this example, the input sequence $\vec{x}$ 505 is first linearly projected to a high-dimensional space, to which positional encodings 515 are added. The output is processed by a sequence of N encoder blocks 520, each of which contains an attention layer 525 and a fully-connected (feed-forward) neural network 530. Finally, the output is passed through a final linear layer 540, producing an output estimate $\vec{y}$ 545 describing the future position.

In more detail, the input sequence $\vec{x}$ 505 is projected to a high-dimensional space by a linear layer 510. Then positional encodings 515 are added to each input embedding to encode the position of each value within the sequence. The core of the Transformer model 535 is composed of a stack of encoder blocks 520. Each encoder block 520 contains a self-attention layer 525 (as discussed above) and a fully-connected neural network 530. Also, in each encoder block 520, normalization layers and/or skip connections may be used to ease optimization. Finally, after the input is processed by a sequence of such encoder blocks 520, a fully-connected layer 540 takes the final encoder output and maps the feature vectors to the output dimension of the predicted value $\vec{y}$ as the output estimate 545.

In an example, the Transformer model 535 may be updated throughout treatment at intervals $\Delta_{update}$. Updating the Transformer model 535 can be performed using a standard gradient-based optimizer with a suitable objective function, such as the mean square error (MSE) or the mean absolute deviation (MAE) between the prediction value (the output of the Transformer model 535) and the corresponding ground truth. Optionally, regularization terms (such as the L2 norm on model weights) may be added to the objective function to combat over-fitting. In a typical configuration, the loss function to minimize can be represented by the following:

$$L = \sum_{i} \|f(\vec{x_i}, \theta) - \vec{y_i}\|^2 + \alpha \|\theta\|^2$$

**(EQUATION 4)**

where $\theta$ are the model parameters and $\alpha$ is a regularization parameter. In other embodiments, additional loss functions promoting the production of smooth predicted position trajectories (e.g. by penalizing differences between predicted positions across sequential time points) may be incorporated into the objective function.

The following paragraphs provide examples of a treatment workflow adapted for performing APM (e.g., with the APM model 430 or a similar APM data processing workflow) with use of a Transformer deep learning model (e.g., Transformer model 535) as the trained AI model 435, including a specific example which considers a sequence of respiratory signals (e.g., multiple breathing cycles) captured in real-time during a radiotherapy treatment session. This sequence of respiratory signals can be used to model long-range interactions.

The following paragraphs also provide examples of a training workflow adapted for developing the trained AI model 435. It will be understood that the following treatment workflow process may be performed and repeated many times (e.g., on an ongoing, real-time basis, to monitor for patient movement) as part of a radiotherapy treatment session for a single patient. The following aspects of training and treatment may also be adapted for use of multiple treatment sessions for a particular patient, or for multiple patients.

**FIG. 6** provides a high-level illustration of a treatment workflow for performing APM (e.g., using the APM model 430), using results of a trained Transformer AI model 640. This treatment workflow includes the capture and processing of real-time data in the form of the latest image 601 and prior images 602, feature extraction 611, 612 for such images 601, 602, identification of the latest and prior spatial information 620, 630 from such images, and the analysis of such spatial information with the Transformer AI model 640. The Transformer AI model 640 is trained to generate (produce) an inferred (estimated) data output, such as in the form of a predicted spatial position. Such spatial position information may be used by a relative motion estimation 650 to determine estimated spatial transformation parameters.

The output of the relative motion estimation 650 may represent relative motion (relative to reference information 410, such as the anatomy depicted in the 3D reference volume), indicating motion provided from translation and/or rotation in the three dimensions. As will be understood, the relative motion may be compared to a reference image and position; thus, the relative motion estimation 650 can be represented by a deviation (e.g., a 3D vector) from the reference image in relative terms, so that the change in absolute terms can be identified.

In further examples, the workflow referenced within FIG. 6 can be performed independently for different structures, using different regions of interest for the tumors and/or organs at risk. Thus, in some examples, different features may be extracted for different anatomical structures; likewise, multiple AI model instances and algorithms may be trained and used to analyze motion of different anatomical structures.

**FIGS. 7A** and **7B** illustrate respective flowcharts for a method of training a generative AI model for estimating anatomic position, and a method of monitoring anatomic position of a human subject using the trained generative AI model.

The flowchart 710 depicts the method of training as including the following operations. Additional data processing aspects discussed above may be integrated into one of more of the following operations.

Operation 711 includes extracting features in image data, from the imaging of multiple human subjects.

Operation 712 includes obtaining sequential temporal data corresponding to observed positions of anatomy in the multiple human subjects. As a result, the training data used in the following training method may include time-ordered sequences of the extracted features (e.g., temporal sequences of observed anatomical positions from a plurality of human subjects).

Operation 713 includes training a generative AI model (e.g., a transformer deep learning neural network) based on the sequential temporal data. Consistent with the examples above, the model may be configured and trained to receive observed position data as input and provide estimated position data as output. Further, the model may be configured and trained to directly or indirectly output transformation parameters that indicate relative motion of a tracked anatomical area (e.g., the area indicated in the received observed position data).

Operation 714 includes providing the trained generative AI model for use in a radiotherapy treatment session. In an example, the input of the trained generative AI model includes observed position data representing observed positions of the tracked anatomical area that are captured over multiple observed breathing cycles in the radiotherapy treatment session. In an example, the trained generative AI model may be used to identify a relative motion of the tracked anatomical area that corresponds to one or more predicted breathing cycles.

Operation 715 includes collecting additional sequential temporal data (e.g., from a particular patient) during the radiotherapy treatment session. Operation 716 includes re-training the generative AI model based on the radiotherapy treatment session, using the additional sequential temporal data (e.g., corresponding to observed positions of a tracked anatomical area of the particular patient).

The flowchart 720 depicts the method of use (e.g., inference) as including the following operations. Additional data processing aspects discussed above may be integrated into one of more of the following operations.

Operation 721 includes extracting features in image data captured during a radiotherapy treatment session for a particular patient, to identify features that represent position information of the particular patient. The position information may be observed (e.g., in real-time) during the radiotherapy treatment session. The position information may be based on image data captured during the radiotherapy treatment session. The position information may be generated by extracting features from multiple images of such image data.

Operation 722 includes providing the position information as an input to a generative AI model that is trained with temporal sequences of observed positions (e.g., in training data). The position information may indicate a position of the tracked anatomical area of the patient in a 3D reference volume (e.g., captured at an earlier time). The generative AI model may be a transformer deep learning neural network model, as discussed in the examples above, but may be provided by other types of trained AI models. The observed positions of anatomy that are used to train the generative AI model may be observed from the patient and multiple other human subjects.

Operation 723 includes determining an estimated position of a tracked anatomical area, at a future time, based on an output of the generative AI model. In an example, the estimated position may be based on relative motion of the tracked anatomical area from translation or rotation in a coordinate space of the 3D reference volume. Here, the output of the generative AI model may provide transformation parameters that indicate the relative motion of the tracked anatomical area of the patient relative to the 3D reference volume. Also in an example, the output of the generative AI model may represent a prediction of respiratory motion to occur at the future time during the radiotherapy treatment session, as the estimated position of the tracked anatomical area of the patient corresponds to the prediction of respiratory motion. Further, the observed positions of the tracked anatomical area of the patient may be captured during multiple observed breathing cycles, as the estimated position of the tracked anatomical area of the patient corresponds to multiple predicted breathing cycles. The tracked anatomical area may specifically correspond to at least one region of interest or at least one organ at risk defined for the radiotherapy treatment session.

Operation 724 includes, optionally, determining the estimated position of the tracked anatomical area, based on captured monitoring signals from one or more sensor(s). Such monitoring signals may include a measurement of respiratory motion observed at a prior time of the radiotherapy treatment session.

Operation 725 includes controlling a radiotherapy treatment session, based on an estimated position of the tracked anatomical area. For instance, controlling may include modifying operation of a radiotherapy machine based on motion of the estimated position of the tracked anatomical area, including one or more of: changing a position of a radiotherapy beam from the radiotherapy machine; changing a shape of a radiotherapy beam from the radiotherapy machine; or gating a radiotherapy beam from the radiotherapy machine.

Operation 726 includes re-training the generative AI model during the radiotherapy treatment session, based on the observed positions. For instance, the generative AI model may be re-trained at a plurality of update intervals during the radiotherapy treatment session, based on the observed positions of the tracked anatomical area of the patient undergoing treatment.

**FIG. 8** illustrates a flowchart 800 of a method of using a trained AI model, for estimating movement in a region of interest, based on the techniques discussed above. For instance, the following features of flowchart 800 may be integrated or adapted with the model usage discussed with reference to FIG. 6.

Operation 810 begins with obtaining image data corresponding to a human subject, on an ongoing basis. This is followed by operation 820, to identify position information of the subject from the image data. This may include position information obtained from one or more orientations, and at multiple times. In some examples, this includes at least a first two-dimensional image that is captured at a first time during the radiotherapy treatment session and a second two-dimensional image that is captured at a second time during the radiotherapy treatment session (e.g., within 300 milliseconds (ms), or according to another time duration which enables real-time processing). As will be understood, there is often system latency due to the time it takes for acquiring an image, operating the motion estimation algorithm, and activating the MLC. However, in some examples, the image data itself can be acquired and processed at a faster frequency (e.g., 200ms) using multiple threads and/or processes (e.g., one process can be processing a 2D image frame while another process performs acquisition of the next frame).

Operation 840 includes analysis of the current position with the trained AI model (e.g., a Transformer model), that has been trained based on the past positions and related movement information. Such a model can be used to estimate a future position, based on the current position.

Operation 850 identifies a movement state of subject, based on an estimated future movement. This may be accompanied by operations (sequentially or in parallel), such as operation 860, which utilizes the radiotherapy workflow (e.g., in the image processing computing system 110) to locate a radiation therapy target within the subject using the identified movement state, or operation 870 to track a radiation therapy target within subject in real-time using the identified movement state.

Operation 880 directs or controls radiation therapy using a treatment machine (e.g., based on image processing computing system 110) to the radiation therapy target according to the identified movement state. It will be understood that a variety of existing approaches for modifying or adapting radiotherapy treatment may occur based on the controlled therapy or identified movement state, once correctly estimated.

**FIG. 9** illustrates a block diagram of an example of a machine 900 on which one or more of the methods as discussed herein can be implemented. In one or more examples, one or more items of the image processing computing system 110 can be implemented by the machine 900. In alternative examples, the machine 900 operates as a standalone device or may be connected (e.g., networked) to other machines. In one or more examples, the image processing computing system 110 can include one or more of the items of the machine 900. In a networked deployment, the machine 900 may operate in the capacity of a server or a client machine in server-client network environment, or as a peer machine in a peer-to-peer (or distributed) network environment. The machine may be a personal computer (PC), server, a tablet, smartphone, a web appliance, edge computing device, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

The example machine 900 includes processing circuitry or processor 902 (e.g., a CPU, a graphics processing unit (GPU), an ASIC, circuitry, such as one or more transistors, resistors, capacitors, inductors, diodes, logic gates, multiplexers, buffers, modulators, demodulators, radios (e.g., transmit or receive radios or transceivers), sensors 921 (e.g., a transducer that converts one form of energy (e.g., light, heat, electrical, mechanical, or other energy) to another form of energy), or the like, or a combination thereof), a main memory 904 and a static memory 906, which communicate with each other via a bus 908. The machine 900 (e.g., computer system) may further include a video display device 910 (e.g., a liquid crystal display (LCD) or a cathode ray tube (CRT)). The machine 900 also includes an alphanumeric input device 912 (e.g., a keyboard), a user interface (UI) navigation device 914 (e.g., a mouse), a disk drive or mass storage unit 916, a signal generation device 918 (e.g., a speaker), and a network interface device 920.

The disk drive unit 916 includes a machine-readable medium 922 on which is stored one or more sets of instructions and data structures (e.g., software) 924 embodying or utilized by any one or more of the methodologies or functions described herein. The instructions 924 may also reside, completely or at least partially, within the main memory 904 and/or within the processor 902 during execution thereof by the machine 900, the main memory 904 and the processor 902 also constituting machine-readable media.

The machine 900 as illustrated includes an output controller 928. The output controller 928 manages data flow to/from the machine 900. The output controller 928 is sometimes called a device controller, with software that directly interacts with the output controller 928 being called a device driver.

While the machine-readable medium 922 is shown in an example to be a single medium, the term "machine-readable medium" may include a single medium or multiple media (e.g., a centralized or distributed database, and/or associated caches and servers) that store the one or more instructions or data structures. The term "machine-readable medium" shall also be taken to include any tangible medium that is capable of storing, encoding or carrying instructions for execution by the machine and that cause the machine to perform any one or more of the methodologies of the present disclosure, or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including by way of example semiconductor memory devices, e.g., Erasable Programmable Read-Only Memory (EPROM), EEPROM, and flash memory devices; magnetic disks such as internal hard disks and removable disks;

magneto-optical disks; and CD-ROM and DVD-ROM disks.

The instructions 924 may further be transmitted or received over a communications network 926 using a transmission medium. The instructions 924 may be transmitted using the network interface device 920 and any one of a number of well-known transfer protocols (e.g., HTTP). Examples of communication networks include a LAN, a WAN, the Internet, mobile telephone networks, Plain Old Telephone (POTS) networks, and wireless data networks (e.g., Wi-Fi and 4G/5G data networks). The term "transmission medium" shall be taken to include any intangible medium that is capable of storing, encoding or carrying instructions for execution by the machine, and includes digital or analog communications signals or other intangible media to facilitate communication of such software.

As used herein, "communicatively coupled between" means that the entities on either of the coupling must communicate through an item therebetween and that those entities cannot communicate with each other without communicating through the item.

Additional Notes

The above detailed description includes references to the accompanying drawings, which form a part of the detailed description. The drawings show, by way of illustration but not by way of limitation, specific embodiments in which the disclosure can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a," "an," "the," and "said" are used when introducing elements of aspects of the disclosure or in the embodiments thereof, as is common in patent documents, to include one or more than one or more of the elements, independent of any other instances or usages of "at least one" or "one or more." In this document, the term "or" is used to refer to a nonexclusive or, such that "A or B" includes "A but not B," "B but not A," and "A and B," unless otherwise indicated.

In the appended claims, the terms "including" and "in which" are used as the plain-English equivalents of the respective terms "comprising" and "wherein." Also, in the following claims, the terms "comprising," "including," and "having" are intended to be open-ended to mean that there may be additional elements other than the listed elements, such that after such a term (e.g., comprising, including, having) in a claim are still deemed to fall within the scope of that claim. Moreover, in the following claims, the terms "first," "second," and "third," and so forth, are used merely as labels, and are not intended to impose numerical requirements on their objects.

Embodiments of the disclosure may be implemented with computer-executable instructions. The computer-executable instructions (e.g., software code) may be organized into one or more computer-executable components or modules. Aspects of the disclosure may be implemented with any number and organization of such components or modules. For example, aspects of the disclosure are not limited to the specific computer-executable instructions or the specific components or modules illustrated in the figures and described herein. Other embodiments of the disclosure may include different computer-executable instructions or components having more or less functionality than illustrated and described herein.

Method examples (e.g., operations and functions) described herein can be machine or computer-implemented at least in part (e.g., implemented as software code or instructions). Some examples can include a computer-readable medium or machine-readable medium encoded with instructions operable to configure an electronic device to perform methods as described in the above examples. An implementation of such methods can include software code, such as microcode, assembly language code, a higher-level language code, or the like (e.g., "source code"). Such software code can include computer-readable instructions for performing various methods (e.g., "object" or "executable code"). The software code may form portions of computer program products. Software implementations of the embodiments described herein may be provided via an article of manufacture with the code or instructions stored thereon, or via a method of operating a communication interface to send data via a communication interface (e.g., wirelessly, over the internet, via satellite communications, and the like).

Further, the software code may be tangibly stored on one or more volatile or non-volatile computer-readable storage media during execution or at other times. These computer-readable storage media may include any mechanism that stores information in a form accessible by a machine (e.g., computing device, electronic system, and the like), such as, but are not limited to, floppy disks, hard disks, removable magnetic disks, any form of magnetic disk storage media, CD-ROMS, magnetic-optical disks, removable optical disks (e.g., compact disks and digital video disks), flash memory devices, magnetic cassettes, memory cards or sticks (e.g., secure digital cards), RAMs (e.g., CMOS RAM and the like), recordable/non-recordable media (e.g., read only memories (ROMs)), EPROMS, EEPROMS, or any type of media suitable for storing electronic instructions, and the like. Such computer-readable storage medium is coupled to a computer system bus to be accessible by the processor and other parts of the OIS.

In an embodiment, the computer-readable storage medium may have encoded a data structure for treatment planning, wherein the treatment plan may be adaptive. The data structure for the computer-readable storage medium may be at least one of a Digital Imaging and Communications in Medicine (DICOM) format, an extended DICOM format, an XML format, and the like. DICOM is an international communications standard that defines the format used to transfer medical image-related data between various types of medical equipment. DICOM RT refers to the communication standards that are specific to radiation therapy.

In various embodiments of the disclosure, the method of creating a component or module can be implemented in software, hardware, or a combination thereof. The methods provided by various embodiments of the present disclosure, for example, can be implemented in software by using standard programming languages such as, for example, C, C++, C#, Java, Python, CUDA programming, and the like; and combinations thereof. As used herein, the terms "software" and "firmware" are interchangeable, and include any computer program stored in memory for execution by a computer.

A communication interface includes any mechanism that interfaces to any of a hardwired, wireless, optical, and the like, medium to communicate to another device, such as a memory bus interface, a processor bus interface, an Internet connection, a disk controller, and the like. The communication interface can be configured by providing configuration parameters and/ or sending signals to prepare the communication interface to provide a data signal describing the software content. The communication interface can be accessed via one or more commands or signals sent to the communication interface.

The present disclosure also relates to a system for performing the operations herein. This system may be specially constructed for the required purposes, or it may comprise a general purpose computer selectively activated or reconfigured by a computer program stored in the computer. The order of execution or performance of the operations in embodiments of the disclosure illustrated and described herein is not essential, unless otherwise specified. That is, the operations may be performed in any order, unless otherwise specified, and embodiments of the disclosure may include additional or fewer operations than those disclosed herein. For example, it is contemplated that executing or performing a particular operation before, contemporaneously with, or after another operation is within the scope of aspects of the disclosure.

In view of the above, it will be seen that the several objects of the disclosure are achieved and other advantageous results attained. Having described aspects of the disclosure in detail, it will be apparent that modifications and variations are possible without departing from the scope of aspects of the disclosure as defined in the appended claims. As various changes could be made in the above constructions, products, and methods without departing from the scope of aspects of the disclosure, it is intended that all matter contained in the above description and shown in the accompanying drawings shall be interpreted as illustrative and not in a limiting sense.

The above description is intended to be illustrative, and not restrictive. For example, the above-described examples (or one or more aspects thereof) may be used in combination with each other. In addition, many modifications may be made to adapt a particular situation or material to the teachings of the disclosure without departing from its scope. While the dimensions, types of materials and coatings described herein are intended to define the parameters of the disclosure, they are by no means limiting and are exemplary embodiments. Many other embodiments will be apparent to those of skill in the art upon reviewing the above description.

Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the invention to which this European patent relates should be determined with reference to the appended claims.

**Claims**

1. A computer-implemented method for monitoring anatomic position of a human subject for a radiotherapy treatment session, the method comprising:

   receiving position information corresponding to observed positions of a tracked anatomical area of a patient, the position information observed during the radiotherapy treatment session;
   providing (722) the position information as an input to a trained generative artificial intelligence, AI, model, wherein the trained generative AI model comprises a transformer deep learning neural network, wherein the transformer uses self-attention, and is trained with temporal sequences of observed anatomical positions from training data; and
   determining (723) an estimated position of the tracked anatomical area of the patient at a future time, based on output of the trained generative AI model.

2.  The method of claim 1, wherein the position information is based on image data captured during the radiotherapy treatment session.

3.  The method of claim 2, the method further comprising:
    generating the position information by extracting features from multiple images of the image data.

4.  The method of claim 2, wherein the position information indicates a position of the tracked anatomical area of the patient in a 3D reference volume, and wherein the estimated position is based on relative motion of the tracked anatomical area from translation or rotation in a coordinate space of the 3D reference volume; and
    optionally, wherein the output of the trained generative AI model provides transformation parameters that indicate the relative motion of the tracked anatomical area of the patient relative to the 3D reference volume.

5.  The method of any of claims 1 to 4, wherein the estimated position is further determined based on monitoring signals captured during the radiotherapy treatment session from one or more sensors, and
    optionally, wherein the monitoring signals include a measurement of respiratory motion observed at a prior time of the radiotherapy treatment session.

6.  The method of any of claims 1 to 5, wherein the output of the trained generative AI model represents a prediction of respiratory motion to occur at the future time during the radiotherapy treatment session, and wherein the estimated position of the tracked anatomical area of the patient corresponds to the prediction of respiratory motion,
    and optionally, wherein the observed positions of the tracked anatomical area of the patient are captured during multiple observed breathing cycles, and wherein the estimated position of the tracked anatomical area of the patient corresponds to multiple predicted breathing cycles.

7.  The method of any of claims 1 to 6, wherein the trained generative AI model is re-trained at a plurality of update intervals during the radiotherapy treatment session, based on the observed positions of the tracked anatomical area of a patient.

8.  The method of any of claims 1 to 7, wherein the observed positions of anatomy used to train the trained generative AI model are observed from the patient and multiple other human subjects.

9.  The method of any of claims 1 to 8, wherein the tracked anatomical area corresponds to at least one region of interest or at least one organ at risk defined for the radiotherapy treatment session.

10. A computer-readable storage medium comprising computer-readable instructions for using a trained generative AI model for monitoring anatomic position of a human subject for a radiotherapy treatment session, wherein the instructions, when executed, cause a computing machine to perform any of the methods of claims 1 to 9.

11. The computer-readable storage medium of claim 10, further comprising controlling the radiotherapy treatment session based on the estimated position of the tracked anatomical area of the patient, and
    optionally wherein controlling the radiotherapy treatment session modifies operation of a radiotherapy machine based on motion caused by the estimated position of the tracked anatomical area, including one or more of:

    changing a position of a radiotherapy beam from the radiotherapy machine;
    changing a shape of a radiotherapy beam from the radiotherapy machine; or
    gating a radiotherapy beam from the radiotherapy machine.

12. A computer-implemented method for training a generative artificial intelligence (AI) model comprising a transformer deep learning neural network for estimating anatomic position, wherein the transformer uses self-attention, the method comprising:

    receiving (712) training data providing temporal sequences of observed anatomical positions in a plurality of human subjects;
    training (713) the generative AI model with the training data, the generative AI model configured to receive observed position data as input and provide estimated position data as output; and
    outputting the trained generative AI model for use in a radiotherapy treatment session.

13. The method of claim 12, wherein the output of the trained generative AI model includes transformation parameters that indicate relative motion of a tracked anatomical area indicated in the observed position data;
and optionally, wherein the input of the trained AI model includes observed position data representing observed positions of the tracked anatomical area that are captured over multiple observed breathing cycles.

**14.** A computer-readable storage medium comprising computer-readable instructions for using a trained generative AI model for monitoring anatomic position of a human subject for a radiotherapy treatment session, wherein the instructions, when executed, cause a computing machine to perform any of the methods of claims 12 or 13.

**Patentansprüche**

**1.** Computerimplementiertes Verfahren zum Überwachen der anatomischen Position eines menschlichen Subjekts für eine Strahlentherapiebehandlungssitzung, wobei das Verfahren umfasst:

Empfangen von Positionsinformationen, die beobachteten Positionen eines verfolgten anatomischen Bereichs eines Patienten entsprechen, wobei die Positionsinformationen während der Strahlentherapiebehandlungssitzung beobachtet werden;
Bereitstellen (722) der Positionsinformationen als Eingabe an ein trainiertes generatives Künstliche Intelligenz-, KI-, Modell, wobei das trainierte generative KI-Modell ein neuronales Transformer-Deep-Learning-Netzwerk umfasst, wobei der Transformer Selbstaufmerksamkeit verwendet und mit zeitlichen Sequenzen beobachteter anatomischer Positionen aus Trainingsdaten trainiert wird; und
Bestimmen (723) einer geschätzten Position des verfolgten anatomischen Bereichs des Patienten zu einer zukünftigen Zeit basierend auf Ausgabe des trainierten generativen KI-Modells.

**2.** Verfahren nach Anspruch 1, wobei die Positionsinformationen auf Bilddaten basieren, die während der Strahlentherapiebehandlungssitzung aufgenommen wurden.

**3.** Verfahren nach Anspruch 2, wobei das Verfahren ferner umfasst:
Generieren der Positionsinformationen durch Extrahieren von Merkmalen aus mehreren Bildern der Bilddaten.

**4.** Verfahren nach Anspruch 2, wobei die Positionsinformationen eine Position des verfolgten anatomischen Bereichs des Patienten in einem 3D-Referenzvolumen angeben, und wobei die geschätzte Position auf relativer Bewegung des verfolgten anatomischen Bereichs aus Translation oder Rotation in einem Koordinatenraum des 3D-Referenzvolumens basiert; und
wobei gegebenenfalls die Ausgabe des trainierten generativen KI-Modells Transformationsparameter bereitstellt, die die relative Bewegung des verfolgten anatomischen Bereichs des Patienten relativ zu dem 3D-Referenzvolumen angeben.

**5.** Verfahren nach einem der Ansprüche 1 bis 4, wobei die geschätzte Position ferner basierend auf Überwachungssignalen bestimmt wird, die während der Strahlentherapiebehandlungssitzung von einem oder mehreren Sensoren aufgenommen werden, und
wobei gegebenenfalls die Überwachungssignale eine Messung der Atembewegung einschließen, die zu einer früheren Zeit der Strahlentherapiebehandlungssitzung beobachtet wurde.

**6.** Verfahren nach einem der Ansprüche 1 bis 5, wobei die Ausgabe des trainierten generativen KI-Modells eine Vorhersage einer Atembewegung repräsentiert, die zu der zukünftigen Zeit während der Strahlentherapiebehandlungssitzung auftreten wird, und wobei die geschätzte Position des verfolgten anatomischen Bereichs des Patienten der Vorhersage einer Atembewegung entspricht,
und wobei gegebenenfalls die beobachteten Positionen des verfolgten anatomischen Bereichs des Patienten während mehrerer beobachteter Atemzyklen aufgenommen werden, und wobei die geschätzte Position des verfolgten anatomischen Bereichs des Patienten mehreren vorhergesagten Atemzyklen entspricht.

**7.** Verfahren nach einem der Ansprüche 1 bis 6, wobei das trainierte generative KI-Modell in einer Vielzahl von Aktualisierungsintervallen während der Strahlentherapiebehandlungssitzung basierend auf den beobachteten Positionen des verfolgten anatomischen Bereichs eines Patienten erneut trainiert wird.

**8.** Verfahren nach einem der Ansprüche 1 bis 7, wobei die beobachteten Positionen der Anatomie, die zum Trainieren des trainierten generativen KI-Modells verwendet werden, von dem Patienten und mehreren anderen menschlichen Subjekten beobachtet werden.

9.  Verfahren nach einem der Ansprüche 1 bis 8, wobei der verfolgte anatomische Bereich mindestens einer Interessensregion oder mindestens einem Organ entspricht, wofür bei der Strahlentherapiebehandlungssitzung ein Risiko definiert ist.

10. Computerlesbares Speichermedium, umfassend computerlesbare Anweisungen zum Verwenden eines trainierten generativen KI-Modells zum Überwachen der anatomischen Position eines menschlichen Subjekts für eine Strahlentherapiebehandlungssitzung, wobei die Anweisungen, wenn sie ausgeführt werden, veranlassen, dass ein Rechengerät eines der Verfahren nach den Ansprüchen 1 bis 9 durchführt.

11. Computerlesbares Speichermedium nach Anspruch 10, ferner umfassend Steuern der Strahlentherapiebehandlungssitzung basierend auf der geschätzten Position des verfolgten anatomischen Bereichs des Patienten, und wobei gegebenenfalls Steuern der Strahlentherapiebehandlungssitzung den Betrieb eines Strahlentherapiegeräts basierend auf Bewegung modifiziert, die durch die geschätzte Position des verfolgten anatomischen Bereichs verursacht wird, einschließlich eines oder mehrerer der Folgenden:

    Ändern einer Position eines Strahlentherapiestrahls von dem Strahlentherapiegerät;
    Ändern einer Form eines Strahlentherapiestrahls von dem Strahlentherapiegerät; oder
    Gating eines Strahlentherapiestrahls von dem Strahlentherapiegerät.

12. Computerimplementiertes Verfahren zum Trainieren eines generativen Künstliche Intelligenz (KI)-Modells, umfassend ein neuronales Transformer-Deep-Learning-Netzwerk zum Schätzen von anatomischer Position, wobei der Transformer Selbstaufmerksamkeit verwendet, wobei das Verfahren umfasst:

    Empfangen (712) von Trainingsdaten, die zeitliche Sequenzen beobachteter anatomischer Positionen in einer Vielzahl von menschlichen Subjekten bereitstellen;
    Trainieren (713) des generativen KI-Modells mit den Trainingsdaten, wobei das generative KI-Modell dazu ausgelegt ist, beobachtete Positionsdaten als Eingabe zu empfangen und geschätzte Positionsdaten als Ausgabe bereitzustellen; und
    Ausgeben des trainierten generativen KI-Modells zur Verwendung in einer Strahlentherapiesitzung.

13. Verfahren nach Anspruch 12, wobei die Ausgabe des trainierten generativen KI-Modells Transformationsparameter einschließt, die relative Bewegung eines verfolgten anatomischen Bereichs angeben, der in den beobachteten Positionsdaten angegeben ist; und wobei gegebenenfalls die Eingabe des trainierten KI-Modells beobachtete Positionsdaten einschließt, die beobachtete Positionen des verfolgten anatomischen Bereichs repräsentieren, die über mehrere beobachtete Atemzyklen aufgenommen werden.

14. Computerlesbares Speichermedium, umfassend computerlesbare Anweisungen zum Verwenden eines trainierten generativen KI-Modells zum Überwachen der anatomischen Position eines menschlichen Subjekts für eine Strahlentherapiebehandlungssitzung, wobei die Anweisungen, wenn sie ausgeführt werden, veranlassen, dass ein Rechengerät eines der Verfahren nach den Ansprüchen 12 bis 13 durchführt.

**Revendications**

1.  Procédé mis en œuvre par ordinateur de surveillance de la position anatomique d'un sujet humain pour une session de traitement de radiothérapie, le procédé comprenant :

    la réception d'informations de position correspondant aux positions observées d'une zone anatomique suivie d'un patient, les informations de position observées pendant la séance de radiothérapie ;
    la fourniture (722) des informations de position en tant qu'entrée dans un modèle d'intelligence artificielle générative entraînée, IA, le modèle d'IA générative entraînée comprenant un réseau neuronal d'apprentissage profond de transformateur, le transformateur utilisant une attention de soi et étant entraîné avec des séquences temporelles de positions anatomiques observées à partir de données d'apprentissage ; et
    la détermination (723) d'une position estimée de la zone anatomique suivie du patient à un moment ultérieur, sur la base de la sortie du modèle d'IA générative entraîné.

**2.** Procédé selon la revendication 1, les informations de position étant basées sur des données d'image capturées pendant la séance de traitement de radiothérapie.

**3.** Procédé selon la revendication 2, le procédé comprenant en outre :
la génération des informations de position par extraction de caractéristiques à partir de multiples images des données d'image.

**4.** Procédé selon la revendication 2, les informations de position indiquant une position de la zone anatomique suivie du patient dans un volume de référence 3D, et la position estimée étant basée sur un mouvement relatif de la zone anatomique suivie à partir d'une translation ou d'une rotation dans un espace de coordonnées du volume de référence 3D ; et
éventuellement, la sortie du modèle d'IA générative entraîné fournissant des paramètres de transformation qui indiquent le mouvement relatif de la zone anatomique suivie du patient par rapport au volume de référence 3D.

**5.** Procédé selon l'une quelconque des revendications 1 à 4, la position estimée étant en outre déterminée sur la base de signaux de surveillance capturés pendant la session de traitement de radiothérapie à partir d'un ou plusieurs capteurs, et
éventuellement, les signaux de surveillance comprenant une mesure du mouvement respiratoire observé à un moment antérieur de la séance de traitement de radiothérapie.

**6.** Procédé selon l'une quelconque des revendications 1 à 5, la sortie du modèle d'IA générative entraîné représentant une prédiction qu'un mouvement respiratoire se produise à l'instant futur pendant la séance de traitement de radiothérapie, et la position estimée de la zone anatomique suivie du patient correspondant à la prédiction du mouvement respiratoire,
et éventuellement, les positions observées de la zone anatomique suivie du patient étant capturées pendant de multiples cycles respiratoires observés, et la position estimée de la zone anatomique suivie du patient correspondant à de multiples cycles respiratoires prédits.

**7.** Procédé selon l'une quelconque des revendications 1 à 6, le modèle d'IA générative entraîné étant réentraîné à une pluralité d'intervalles de mise à jour pendant la séance de traitement de radiothérapie, sur la base des positions observées de la zone anatomique suivie d'un patient.

**8.** Procédé selon l'une quelconque des revendications 1 à 7, les positions observées de l'anatomie utilisées pour entraîner le modèle d'IA générative entraîné étant observées à partir du patient et de multiples autres sujets humains.

**9.** Procédé selon l'une quelconque des revendications 1 à 8, la zone anatomique suivie correspondant à au moins une région d'intérêt ou à au moins un organe à risque défini pour la séance de traitement de radiothérapie.

**10.** Support de stockage lisible par ordinateur comprenant des instructions lisibles par ordinateur pour utiliser un modèle d'IA générative entraîné pour surveiller la position anatomique d'un sujet humain pour une session de traitement de radiothérapie, les instructions, lorsqu'elles sont exécutées, amenant une machine informatique à réaliser l'un quelconque des procédés selon les revendications 1 à 9.

**11.** Support de stockage lisible par ordinateur selon la revendication 10, comprenant en outre la commande de la session de traitement de radiothérapie sur la base de la position estimée de la zone anatomique suivie du patient, et éventuellement, la commande de la séance de traitement de radiothérapie modifiant le fonctionnement d'une machine de radiothérapie en fonction du mouvement provoqué par la position estimée de la zone anatomique suivie, comprenant le ou les des éléments suivants :

la modification de la position d'un faisceau de radiothérapie à partir de la machine de radiothérapie ;
la modification de la forme d'un faisceau de radiothérapie à partir de la machine de radiothérapie ; ou
la synchronisation d'un faisceau de radiothérapie à partir de la machine de radiothérapie.

**12.** Procédé mis en œuvre par ordinateur d'apprentissage d'un modèle d'intelligence artificielle générative (IA) comprenant un réseau neuronal d'apprentissage profond de transformateur pour estimer une position anatomique, le transformateur utilisant une attention de soi, le procédé comprenant :

la réception (712) des données d'apprentissage fournissant des séquences temporelles de positions anatomiques observées chez une pluralité de sujets humains ;

l'apprentissage (713) du modèle d'IA générative avec les données d'apprentissage, le modèle d'IA générative étant configuré pour recevoir des données de position observées en tant qu'entrée et fournir des données de position estimées en tant que sortie ; et

la production du modèle d'IA générative entraînée pour une séance de radiothérapie.

13. Procédé selon la revendication 12, la sortie du modèle d'IA générative entraîné comprenant des paramètres de transformation qui indiquent un mouvement relatif d'une zone anatomique suivie indiquée dans les données de position observées ;

et éventuellement, l'entrée du modèle IA entraîné comprenant des données de position observées représentant des positions observées de la zone anatomique suivie qui sont capturées sur de multiples cycles respiratoires observés.

14. Support de stockage lisible par ordinateur comprenant des instructions lisibles par ordinateur pour utiliser un modèle d'IA générative entraîné pour surveiller la position anatomique d'un sujet humain pour une session de traitement de radiothérapie, les instructions, lorsqu'elles sont exécutées, amenant une machine informatique à réaliser l'un quelconque des procédés selon les revendications 12 ou 13.

FIG. 1

**FIG. 2A**

**FIG. 2B**

**FIG. 3**

*FIG. 4*

**FIG. 5**

**FIG. 6**

710

711 —◯ 
┌─────────────────────────────────────────────────────────┐
│            EXTRACT FEATURES IN IMAGE DATA,              │
│        FROM IMAGING OF MULTIPLE HUMAN SUBJECTS          │
└─────────────────────────────────────────────────────────┘

712 —◯
┌─────────────────────────────────────────────────────────┐
│            OBTAIN SEQUENTIAL TEMPORAL DATA             │
│   CORRESPONDING TO OBSERVED POSITIONS OF ANATOMY       │
│           IN THE MULTIPLE HUMAN SUBJECTS               │
└─────────────────────────────────────────────────────────┘

713 —◯
┌─────────────────────────────────────────────────────────┐
│    TRAIN GENERATIVE AI MODEL (E.G., TRANSFORMER DNN)   │
│           BASED ON SEQUENTIAL TEMPORAL DATA            │
└─────────────────────────────────────────────────────────┘

714 —◯
┌─────────────────────────────────────────────────────────┐
│            PROVIDE TRAINED GENERATIVE AI MODEL         │
│         FOR USE IN RADIOTHERAPY TREATMENT SESSION      │
└─────────────────────────────────────────────────────────┘

715 —◯
┌─────────────────────────────────────────────────────────┐
│         COLLECT ADDITIONAL SEQUENTIAL TEMPORAL DATA    │
│          DURING THE RADIOTHERAPY TREATMENT SESSION     │
└─────────────────────────────────────────────────────────┘

716 —◯
┌─────────────────────────────────────────────────────────┐
│            RE-TRAIN THE GENERATIVE AI MODEL           │
│      BASED ON THE RADIOTHERAPY TREATMENT SESSION,      │
│      USING THE ADDITIONAL SEQUENTIAL TEMPORAL DATA     │
└─────────────────────────────────────────────────────────┘

*FIG. 7A*

— 720

721 — EXTRACT POSITION INFORMATION (FEATURES) FROM IMAGE DATA CAPTURED DURING RADIOTHERAPY TREATMENT SESSION

722 — PROVIDE POSITION INFORMATION AS INPUT TO GENERATIVE AI MODEL TRAINED WITH SEQUENCES OF OBSERVED POSITIONS

723 — DETERMINE ESTIMATED POSITION OF TRACKED ANATOMICAL AREA, BASED ON OUTPUT OF GENERATIVE AI MODEL

724 — DETERMINE ESTIMATED POSITION OF TRACKED ANATOMICAL AREA, BASED ON CAPTURED MONITORING SIGNALS FROM SENSOR(S)

725 — CONTROL RADIOTHERAPY TREATMENT SESSION BASED ON ESTIMATED POSITION OF TRACKED ANATOMICAL AREA

726 — RE-TRAIN GENERATIVE AI MODEL DURING RADIOTHERAPY TREATMENT SESSION, BASED ON OBSERVED POSITIONS

*FIG. 7B*

800

820 — CAPTURE IMAGE DATA (ONGOING)

830 — IDENTIFY POSITION INFORMATION
(PAST POSITIONS, CURRENT POSITION)
OF SUBJECT FROM IMAGE DATA

840 — USE TRAINED ARTIFICIAL INTELLIGENCE (E.G. TRANSFORMER) MODEL
TO ESTIMATE FUTURE POSITION FROM CURRENT POSITION

850 — IDENTIFY MOVEMENT STATE OF SUBJECT,
BASED ON ESTIMATED FUTURE MOVEMENT

860 — LOCATE A RADIATION THERAPY
TARGET WITHIN SUBJECT
USING THE IDENTIFIED STATE

870 — TRACK A RADIATION THERAPY
TARGET WITHIN SUBJECT IN REAL-TIME
USING THE IDENTIFIED STATE

880 — DIRECT RADIATION THERAPY, USING A
TREATMENT DEVICE, TO TARGET ACCORDING
TO THE IDENTIFIED STATE

*FIG. 8*

_900_

PROCESSOR _902_

INSTRUCTIONS _924_

MAIN MEMORY _904_

INSTRUCTIONS _924_

STATIC MEMORY _906_

INSTRUCTIONS _924_

SENSORS _921_

NETWORK INTERFACE DEVICE _920_

NETWORK _926_

BUS / INTERCONNECT _908_

DISPLAY DEVICE _910_

INPUT DEVICE _912_

UI NAVIGATION DEVICE _914_

MASS STORAGE _916_

MACHINE-READABLE MEDIUM _922_

INSTRUCTIONS _924_

SIGNAL GENERATION DEVICE _918_

OUTPUT CONTROLLER _928_

_FIG. 9_

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021290977 A1

- US 2021046327 A1